# EUROPEAN PATENT APPLICATION

(11) **EP 4 641 201 A2**
(43) Date of publication of application: **29.10.2025**
(21) Application number: 25187635.5
(22) Date of filing: 25.05.2023
(51) Int. Cl.: G01N 33/574

(54) **COMPOSITIONS AND METHODS FOR MODULATION OF TUMOR SUPPRESSORS AND ONCOGENES**

(30) Priority: 25.05.2022 US 202263345756 P
(62) Divisional of application: 23734854.5
(71) Applicant: Flagship Pioneering Innovations VII, LLC, Cambridge, MA 02142 (US)
(72) Inventor: MANI, Vinidhra, Cambridge (US); WEINSTEIN, Erica, Gabrielle, Cambridge (US); ECHELARD, Yann, Paul Guy Regis, Cambridge (US); KAHVEJIAN, Avak, Cambridge (US); PENDERGRAST, Patrick, Shannon, Cambridge (US); OUSPENSKAIA, Tamara, Cambridge (US); BILLINGS, Gabriel, Humphrey, Cambridge (US); HAMSANATHAN, Shruthi, Cambridge (US); FANG, Tao, Cambridge (US)
(74) Representative: Maiwald GmbH

(57) **Abstract**

The disclosure provides, in various embodiments, compositions, such as polypeptides, polynucleotides, gene editing system, small molecules, vectors or host cells, that comprises and/or modulate expression or activity of cancer-associated proteins. The disclosure also provides, in various embodiments, methods of treating cancer using an agent that comprises and/or modulates expression or activity of a cancer-associated protein and methods of identifying said agent.

## Description

### INCORPORATION BY REFERENCE OF MATERIAL

This application claims the benefit of U.S. Provisional Application No. 63/345,756, filed on May 25, 2022. The entire teachings of the above application are incorporated herein by reference.

### INCORPORATION BY REFERENCE OF MATERIAL IN XML

This application incorporates by reference the Sequence Listing contained in the following eXtensible Markup Language (XML) file being submitted concurrently herewith:
a) File name: 57081026002.xml; created May 23, 2023, 54,371,388 Bytes in size.

### BACKGROUND

Cancer is the second leading cause of death globally, accounting for an estimated 9.6 million deaths, or one in six deaths, in 2018 (www.who.int/health-topics/cancer). Cancer is caused by harmful genomic changes such as mutations that alter gene function and contribute to the malignant behavior of cancer cells. Considering the prevalence of cancer globally, and the limited efficacy of currently available therapeutics, there is a critical need to identify additional and novel therapeutic targets for the treatment of different cancers.

### SUMMARY

The disclosure provided herein is based, in part, on the identification of non-canonical protein targets (e.g., proteins encoded by non-canonical open reading frames (ORFs)) for the treatment of cancer.

In one aspect, the present disclosure relates to an agent that comprises and/or modulates (e.g., increases or decreases) the expression and/or activity of a target protein identified herein (e.g., a target protein listed in the Sequence Listing, in Table A, or a variant of the foregoing). In some embodiments, the agent comprises a target protein identified herein (e.g., a target protein listed in the Sequence Listing, in Table A, or a variant of the foregoing). In certain embodiments, the agent modulates (e.g., increases or decreases) the expression and/or activity of a target protein identified herein (e.g., a target protein listed in the Sequence Listing, in Table A, or a variant of the foregoing). In some embodiments, the agent comprises, consists essentially of, or consists of a polypeptide, a polynucleotide, a gene editing system, a small molecule, or a cell (e.g., a cell therapy). The agent can be an inhibitor or an activator of a target protein identified herein. In some embodiments, the agent modulates the expression of a target protein identified herein. In some embodiments, the agent modulates the activity of a target protein identified herein.

In another aspect, the disclosure provides a pharmaceutical composition comprising a target protein identified herein, and a pharmaceutically acceptable carrier.

In another aspect, the disclosure provides a pharmaceutical composition comprising an agent that modulates the expression or activity of a target protein identified herein, and a pharmaceutically acceptable carrier.

In other aspects, the disclosure relates to a polynucleotide encoding a polypeptide described herein, an expression vector comprising a polynucleotide encoding a polypeptide described herein, and a host cell comprising a polynucleotide encoding a polypeptide described herein.

In another aspect, the disclosure provides a method of detecting a cancer or determining a likelihood of developing cancer in a subject, comprising quantifying an expression or activity of a target protein in a sample from the subject, wherein the level of expression or activity of the target protein in the sample is indicative of the likelihood of developing cancer in the subject.

In another aspect, the disclosure provides a method of preparing a sample that is useful for determining a likelihood of developing cancer in a subject, comprising:
a) obtaining or having obtained a sample from the subject;
b) adding a protease inhibitor, a control peptide, a standard peptide, or a combination thereof to the sample to prepare a sample that is useful for detecting a likelihood of developing cancer; and
c) quantifying an expression or activity of a target protein in the sample prepared in step b).

In some embodiments, the method further comprises treating a subject who is predicted to have a likelihood of developing cancer, comprising administering to the subject an effective amount of an agent that comprises and/or modulates the expression or activity of the target protein identified herein, or a pharmaceutical composition comprising the agent.

In another aspect, the disclosure provides a method of treating a cancer in a subject in need thereof (e.g., a human subject having a cancer), comprising administering to the subject an effective amount of an agent that comprises and/or modulates the expression or activity of a target protein identified herein, or a pharmaceutical composition comprising the agent.

In another aspect, the disclosure provides a method of modulating the expression or activity of a target protein identified in the Sequence Listing, in Table A, or a variant of the foregoing in a cell (e.g., a cancer cell, such as a cancer cell in a subject), comprising contacting the cell (e.g., *in vitro, ex vivo,* or *in vivo*) with an agent that comprises and/or modulates the expression or activity of a target protein identified herein, or a pharmaceutical composition comprising the agent.

In another aspect, the disclosure provides a method of identifying an agent that modulates the expression or activity of a target protein identified herein, comprising:
a) contacting the target protein with an agent; and
b) determining whether the agent modulates the expression or activity of the target protein,
wherein a difference in the expression or activity of the target protein that has been contacted with the agent compared to a reference for the expression or activity of the target protein indicates that the agent modulates the expression or activity of the target protein.

### BRIEF DESCRIPTION OF THE DRAWINGS

The foregoing will be apparent from the following more particular description of example embodiments, as illustrated in the accompanying drawings in which like reference characters refer to the same parts throughout the different views. The drawings are not necessarily to scale, emphasis instead being placed upon illustrating embodiments.

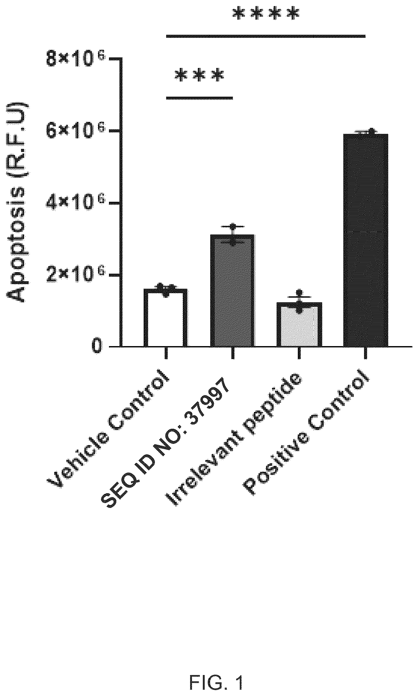

FIG. 1 shows a bar graph showing relative apoptosis in HCT-116 cells treated with vehicle control, SEQ ID NO: 37997 protein, staurosporine (positive control) and irrelevant peptide. * pvalue <0.05, *** pvalue <0.001, ****pvalue <0.0001 vs. vehicle control, one way ANOVA, Dunnett's multiple comparison test.

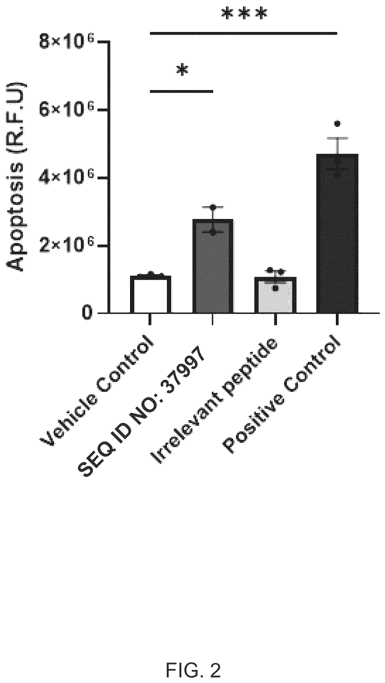

FIG. 2 shows a bar graph showing relative apoptosis in U2OS cells treated with vehicle control, SEQ ID NO: 37997 protein, staurosporine (positive control) and irrelevant peptide. * pvalue <0.05, *** pvalue <0.001, ****pvalue <0.0001 vs. vehicle control, one way ANOVA, Dunnett's multiple comparison test.

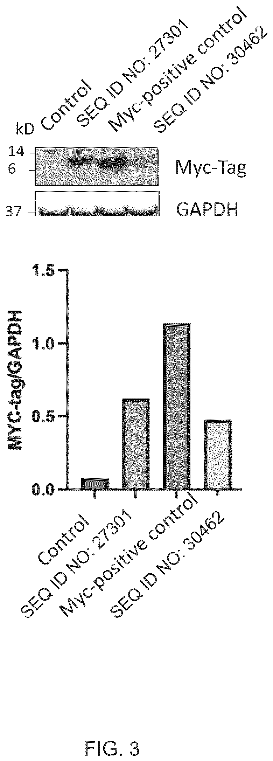

FIG. 3 shows target proteins SEQ ID NO: 27301 and SEQ ID NO: 30462, and myc-positive control are over-expressed in stable cell lines. Left: Western blot of 50 µg of protein extract from stable cell lines with empty vector (Control Vector), target protein SEQ ID NO: 27301, SEQ ID NO: 30462, and myc-positive control. Left (top): anti-myc-tag antibody immunoblot. Left (bottom): anti-GAPDH (loading control). Right: graph of relative expression of control, SEQ ID NO: 27301, SEQ ID NO: 30462, and myc-positive control normalized to GAPDH.

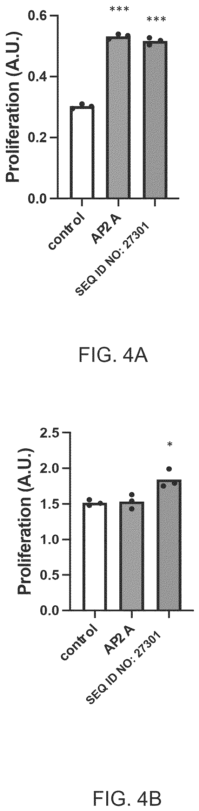

FIGs. 4A and 4B show SEQ ID NO: 27301 over-expression increases proliferation of HCT 116 colon cancer cell line. The results of a WST-1 proliferation assay on HCT116 cells stably expressing Target Protein and control vectors under low serum (FIG. 4A) and high serum (FIG. 4B) conditions. Compared to Empty Vector (control) published oncoprotein AP2A significantly increases proliferation in the low serum condition, but not the high serum condition. However, the target protein SEQ ID NO: 27301 significantly increases proliferation under both low and high conditions.

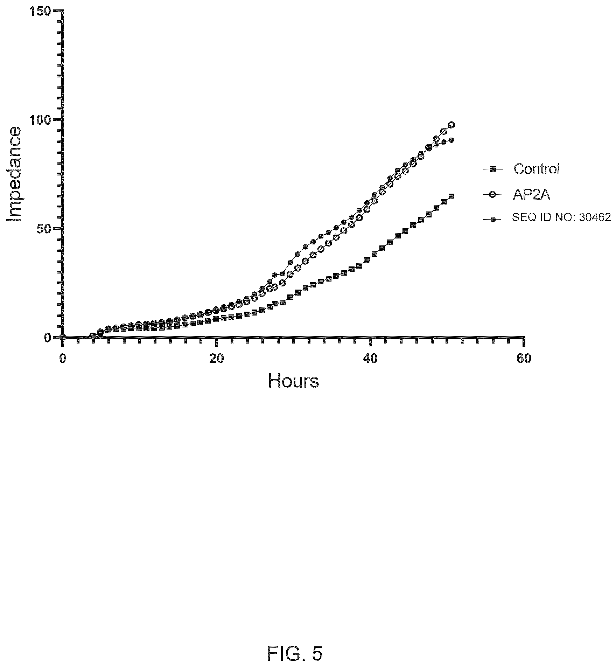

FIG. 5 shows SEQ ID NO: 30462 over-expression increases proliferation of HCT116 colon cancer cell line over time. The results of a Maestro Z impedance-based assay on HCT116 cells stably expressing Target Protein and control vectors. Compared to Empty Vector (control) published oncoprotein AP2A significantly increases proliferation. The target protein, SEQ ID NO: 30462, significantly increases proliferation.

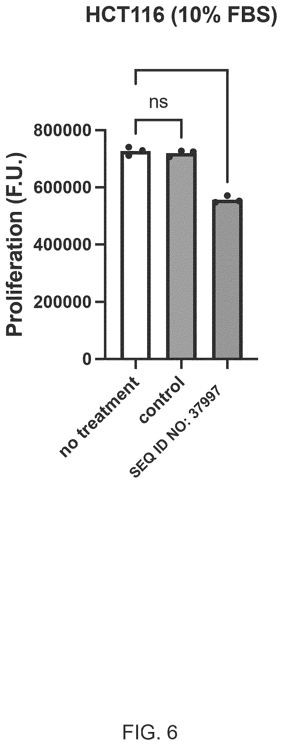

FIG. 6 shows SEQ ID NO: 37997 protein decreases proliferation of HCT116 colon cancer cell line. The results of a WST-1 proliferation assay on HCT116 cells treated with test peptide and control peptide in the presence of 10% serum. Compared to an irrelevant peptide or untreated cells, the target protein significantly decreases proliferation over the course of 48 hours.

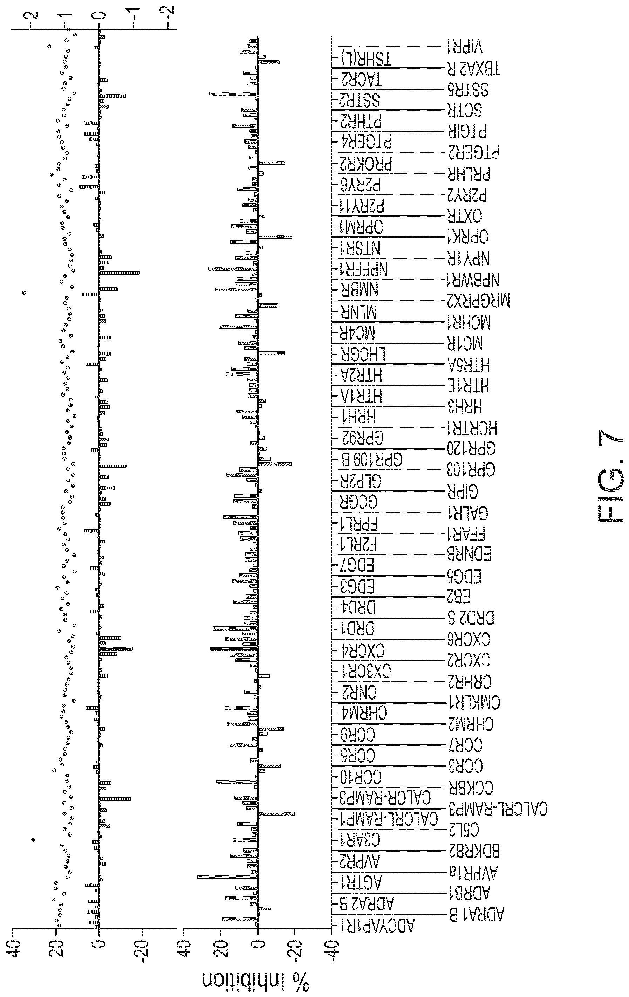

FIG. 7 shows a bar graph showing agonism presented as % activity (*top*) and antagonism presented as % inhibition (*bottom*) for a pool of 9 peptides, including target proteins SEQ ID NO: 30949 and SEQ ID NO: 34229 tested against 168 GPCR targets. % activity refers to β-arrestin recruitment compared to a known agonist at EC80 concentration. % inhibition refers to decrease in β-arrestin recruitment induced by agonist at EC80 concentration. CXCR4 (*black*) showed a significant GPCR antagonist activity, as evidenced by the percent inhibition and suppression of agonist activity against the peptide pool. C3AR1 (*black*) showed a strong agonism when compared to a known agonist and demonstrated a significant fold change with respect to basal activity (*black dot*)*.*

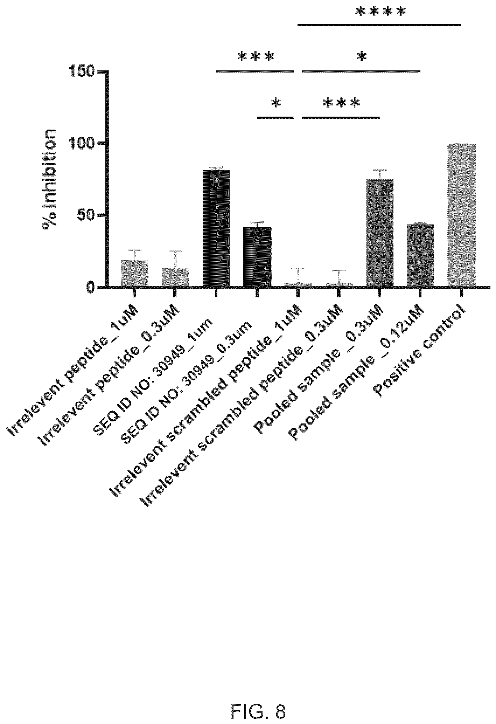

FIG. 8 shows a bar graph showing % inhibition for the target protein, SEQ ID NO: 30949 tested on PathHunter^{®} β-Arrestin cell line for CXCR4 human chemokine GPCR antagonist assay. Target protein SEQ ID NO: 30949, *in black* showed significant inhibition (~80%) at both 1µM and 0.3µM when compared to Irrelevant peptide, irrelevant scrambled peptide, peptide pool with irrelevant peptides and target protein, SEQ ID NO: 30949 in *gray.* * pvalue <0.05, *** pvalue <0.001, ****pvalue <0.0001 vs. irrelevant scrambled peptide_1uM, one way ANOVA, Dunnett's multiple comparison test.

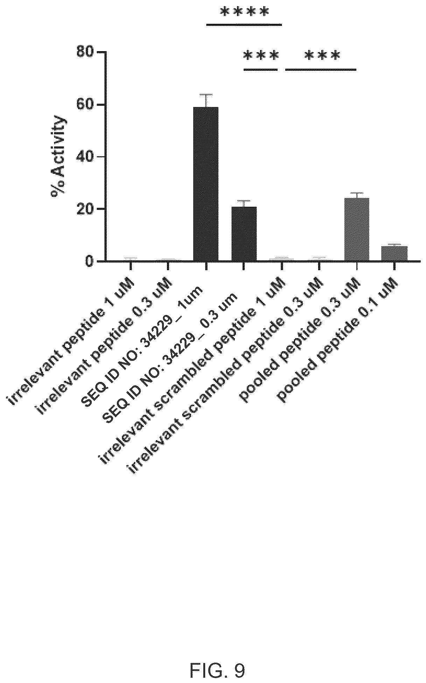

FIG. 9 shows a bar graph showing % activity for the target protein, SEQ ID NO: 34229 tested on PathHunter^{®} β-Arrestin cell line for C3AR1 human complement GPCR antagonist assay. Target protein SEQ ID NO: 34229, *in black* showed significant activity at both 1µM and 0.3µM when compared to Irrelevant peptide, irrelevant scrambled peptide, peptide pool with irrelevant peptides and target protein, SEQ ID NO: 34229 in *gray.* % activity for C3AR1 is with respect to known agonist, C3A Receptor Agonist * pvalue <0.05, *** pvalue <0.001, ****pvalue <0.0001 vs. irrelevant scrambled peptide_1uM, one way ANOVA, Dunnett's multiple comparison test.

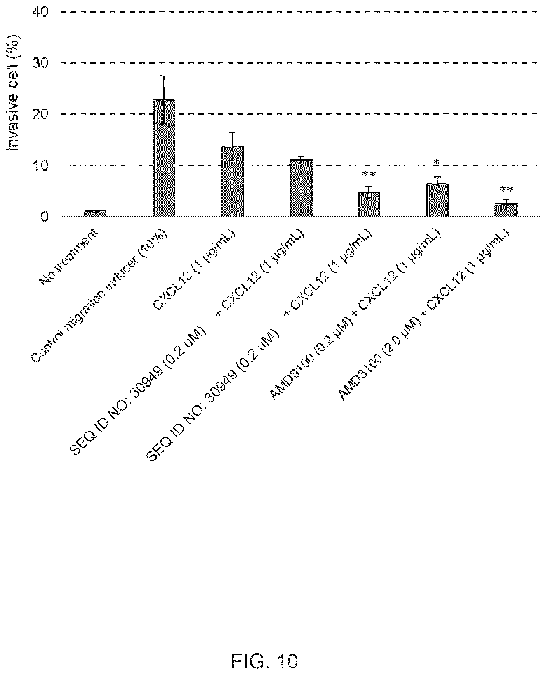

FIG. 10 shows quantification of the chemotactic response of NAMALWA cells migrating towards SEQ ID NO: 30949. Cells were allowed to migrate for 24 hours in the absence or presence of SEQ ID NO: 30949 or SDF-1 (CXCL-12) in the lower chamber of trans-well plates. Migration Inducer was used as positive control and ADM3100 was used as negative control for chemotaxis. The data are presented as the mean ± standard error of the mean (SEM) of the three independent experiments, each performed in triplicate. Statistical analysis was performed using a two-tailed t-test, *p < 0.05.

### DETAILED DESCRIPTION

A description of example embodiments follows.

### Target Proteins

In one aspect, the disclosure provides a target protein identified herein. As used herein, the expressions "target protein identified herein" and "target protein of the disclosure" includes both the polypeptides disclosed in the Sequence Listing and the peptides disclosed in Table A herein. The target protein can be produced recombinantly (e.g., via DNA or mRNA) or synthetically.

In some embodiments, the target protein is a tumor suppressor (e.g., SEQ ID NO: 37997). In some embodiments, the target protein is an oncogene (e.g., SEQ ID NOS: 33586, 36829, and 38556). The target protein can be expressed on a cancer cell (e.g., a metastatic cancer cell), in the tumor microenvironment (e.g., on a stromal cell), or on a non-malignant cell (e.g., an immune cell).

In some embodiments, the target protein is an intracellular protein. In some embodiments, the target protein is an extracellular protein (e.g., a secreted protein). In certain embodiments, the target protein is a transmembrane protein. In particular embodiments, the target protein is membrane bound and extracellular, but is not transmembrane. In more particular embodiments, the target protein is embedded in a membrane, but is not transmembrane.

In various embodiments, the target protein is a protein in the Sequence Listing or Table A. In various embodiments, the target protein is a protein comprising an amino acid sequence set forth in the Sequence Listing or Table A. In some embodiments, the target protein consists of an amino acid sequence set forth in the Sequence Listing or Table A. In some embodiments, the target protein comprises an amino acid sequence having one amino acid substitution relative to an amino acid sequence set forth in the Sequence Listing or Table A, wherein the substitution is substitution of an N-terminal residue in an amino acid sequence in the Sequence Listing or Table A with a methionine (Met) residue. In some embodiments, the target protein consists of an amino acid sequence having one amino acid substitution relative to an amino acid sequence set forth in the Sequence Listing or Table A, wherein the substitution is substitution of an N-terminal residue in an amino acid sequence in the Sequence Listing or Table A with a methionine (Met) residue. In some embodiments, the target protein comprises an amino acid sequence set forth in the Sequence Listing or Table A and further comprises a methionine (Met) residue at its N-terminus. In some embodiments, the target protein consists of an amino acid sequence set forth in the Sequence Listing or Table A and a methionine (Met) residue at its N-terminus.

**Table A. Target Peptides of the Disclosure**

| **SEQ ID NO:** | **Sequence** |
|---|---|
| 21374 | MKR |
| 30784 | RMR |
| 30793 | MVE |
| 30848 | MPL |
| 30889 | MLP |
| 30908 | LVT |
| 30956 | MGT |
| 31100 | MCD |
| 31225 | MIT |
| 31281 | MTK |
| 31418 | VLE |
| 31491 | MNV |
| 31703 | MGM |
| 31738 | MQI |
| 31850 | IGR |
| 31953 | VDE |
| 32091 | MSL |
| 32188 | VAV |
| 32259 | MKQ |
| 32408 | MDA |
| 32427 | LGC |
| 32443 | MKT |
| 32481 | MPR |
| 32555 | IHD |
| 32564 | LHS |
| 32568 | IAG |
| 32611 | MQR |
| 32616 | KTS |
| 32635 | IHT |
| 32714 | TML |
| 32723 | MTD |
| 32724 | MTD |
| 33011 | TRT |
| 33089 | LSK |
| 33096 | MPA |
| 33104 | LAV |
| 33221 | MKD |
| 33287 | LDY |
| 33342 | MPP |

Certain of the target proteins in the Sequence Listing and Table A have been identified as being differentially expressed (e.g., upregulated or downregulated) in a disease state (e.g., a cancer, a pre-cancerous condition) compared to a reference state (e.g., normal state) such that modulation of the level and/or activity of the target protein acts to treat, ameliorate, and/or prevent the development of the disease.

As used herein, the term "differential expression," refers to at least one recognizable difference in protein expression. It may be a quantitatively measurable, semi-quantitatively estimable or qualitatively detectable difference in protein expression. Thus, a differentially expressed protein, or "DEP," may have a higher expression level in a reference state (e.g., normal state) than in a disease state, in which the DEP has a lower expression level or is not expressed at all. Conversely, a DEP may have a higher expression level in a disease state than in a reference state (e.g., normal state), in which the DEP has a lower expression level or is not expressed at all. Further, expression may be regarded as differential if the DEP is recognizably changed (*e.g*., mutated) between two states under comparison. Recognizable changes can include amino acid substitutions, insertions, and/or deletions, including N- and C-terminal truncations, as well as modifications (e.g., post-translational modifications).

As used herein, the term "reference" refers to a standard used for comparison purpose(s). A person skilled in the art can select an appropriate reference for a particular comparison purpose(s). Thus, for example, a reference for a disease state may be a normal, healthy state; a reference for a mutated protein may be the non-mutated protein; a reference for a disease treatment may be no treatment or may be a standard of care treatment. In some embodiments, particularly embodiments involving methods of identifying an agent that modulates the expression and/or activity of a target protein, the reference is the activity and/or expression of the target protein in the absence of the agent. In some embodiments, a reference is based on a predetermined level, e.g., based on functional expression or empirical assays. In some embodiments, a reference obtained from one cell, sample or subject (e.g., a cell or sample from a healthy subject, a subject that does not have a particular disease; a healthy subject, a subject who does not have the particular disease). In some embodiments, a reference is obtained from more than one (e.g., a population of) cell, sample or subject (e.g., a cell or sample from a healthy subject, a subject that does not have a particular disease; a healthy subject, a subject who does not have a particular disease), such as 2, 3, 4, 5, 10, 20, 30, 50, 100 or more, or a statistically significant number of cells, samples or healthy subjects. A reference obtained from more than one cell, sample or subject can be represented as a statistic (e.g., an average or median).

In some embodiments, the target protein has an expression level in a disease (e.g., as determined from a sample from a cell or tissue of a subject having the disease) that is at least about 0.5-fold higher, e.g., at least about: 0.6-, 0.7-, 0.8-, 0.9-, 1.0-, 1.1-, 1.2-, 1.3-, 1.4-, 1.5-, 1.6-, 1.7-, 1.8-, 1.9-, 2.0-, 2.5-, 3-, 3.5-, 4-, 5-, 6-, 7-, 8-, 9- or 10-fold higher (e.g., 50 fold higher, 100-fold higher) than the target protein expression level in a reference (e.g., a sample from a cell or tissue of a subject who does not have the disease).

In some embodiments, the target protein has an expression level in a disease (e.g., as determined from a sample comprising or obtained from a cell or tissue of a subject having the disease) that is at least about 0.5-fold lower, e.g. at least about: 0.6-, 0.7-, 0.8-, 0.9-, 1.0-, 1.1-, 1.2-, 1.3-, 1.4-, 1.5-, 1.6-, 1.7-, 1.8-, 1.9-, 2.0-, 2.5-, 3-, 3.5-, 4-, 5-, 6-, 7-, 8-, 9- or 10-fold lower (e.g., 50-fold lower, 100-fold lower) than the target protein expression level in a reference (e.g., a sample from a cell or tissue of a subject who does not have the disease). In some embodiments, the target protein is not expressed, or is expressed at an undetectable level, in a disease (e.g., as determined from a sample comprising or obtained from a cell or tissue of a subject having the disease).

Non-limiting examples of (biological) samples include blood, blood components (e.g., serum or plasma), urine, saliva, amniotic fluid, cerebrospinal fluid, tissue (e.g., a biopsy or microbiopsy), pancreatic fluid, chorionic villus sample, and cells, etc., isolated from a subject.

In some embodiments, the target protein is translated from a non-coding RNA. In some embodiments, the non-coding RNA is a long intergenic non-coding RNA (lincRNA). In certain embodiments, the non-coding RNA is a long noncoding RNA (lncRNA). In some embodiments, the non-coding RNA is a microRNA (miRNA or miR).

In some embodiments, the target protein is translated from a non-exonic element in an unprocessed precursor mRNA (pre-mRNA). In some embodiments, the non-exonic element is an intron in a pre-mRNA. In some embodiments, the non-exonic element is a 5'-untranslated region (5'-UTR) in a pre-mRNA. In some embodiments, the non-exonic element is a 3'-untranslated region (3'-UTR) in a pre-mRNA.

In some embodiments, the target protein has a length of 2,000 amino acids or less, e.g., 1000 amino acids or less, 750 amino acids or less, 500 amino acids or less, 250 amino acids or less, 150 amino acids or less or 100 amino acids or less. In some embodiments, the target protein has a length of 7 amino acids or more, e.g., 8, 9, 10, 15, 18, 25, 50, 75 or 100 amino acids or more. In certain embodiments, the target protein has a length of from about 50 to about 200 amino acids, e.g., from about 100 to about 150 amino acids. In particular embodiments, the target protein has a length of 7 amino acids or more. In more particular embodiments, the target protein has a length of about 18 amino acids.

Certain of the target proteins disclosed herein (e.g., SEQ ID NO: 30949; SEQ ID NO: 34229) have been identified as modulators of G protein-coupled receptors (GPCRs) (see, e.g., Example 9). Therefore, in some embodiments, a target protein is a modulator of one or more GPCRs, such as, e.g., the GPCRs CXCR4 or C3AR1. In some embodiments, a target protein is an agonist of one or more GPCRs. In some embodiments, a target protein is an antagonist of one or more GPCRs. In some embodiments, a target protein is a direct modulator of one or more GPCRs, such as a ligand for one or more GPCRs. In some embodiments, a target protein is an indirect modulator of one or more GPCRs.

The expression and/or activity of various GPCRs have been linked to different diseases/disorders, conditions and indications, including those set forth in Table B (see, e.g., Kenakin, T., Biased Receptor Signaling in Drug Discovery, Pharmacol Rev 71:267-315, April 2019; Harmar, A.J., et al., IUPHAR-DB: the IUPHAR database of G protein-coupled receptors and ion channels, Nucleic Acids Research, 2009, Vol. 37; and Davenport AP, Scully CCG, de Graaf C, Brown AJH, and Maguire JJ. Advances in therapeutic peptides targeting G protein-coupled receptors. Nat Rev Drug Discov. 2020 Jun.19(6):389-413; the contents of each are incorporated herein by reference in their entirety). Accordingly, in some embodiments, a target protein disclosed herein, which is a modulator of a GPCR, is useful for treating and/or diagnosing one or more diseases/disorders, conditions and/or indications, such as cancer or a pre-cancerous condition, or any of the diseases/disorders, conditions and indications listed in Table B, which are known to be associated with GPCR expression and/or activity.

**Table B. GPCRs Associated with Disease**

| **Disease group** | **GPCRs Associated with Disease Group** |
|---|---|
| acute disease | ADRB1, P2RY12, AGTR1 |
| breast disease | GNRHR |
| cancer or benign tumor | ADRB2, ADRA1A, TSHR, SSTR3, SSTR1, GNRHR, CXCR4, CASR, GCGR, ADRA1D, SSTR4, ADRA1B, OPRM1, SMO, TACR1, OPRK1, SSTR2, ADRB1, MC1R, SSTR5 |
| cardiovascular disease | ADRB3, OPRD1, ADRB2, ADRA1A, AGTR1, ADORA1, TSHR, HTR1D, PTGIR, HTR4, DRD2, F2R, HTR1B, FZD4, EDNRB, ADRA1D, ADORA2A, HTR1F, ADORA3, ADORA2B, CELSR1, CALCRL, ADRA1B, BDKRB2, OPRM1, ADRA2B, GLP1R, AVPR1A, P2RY12, OPRK1, PTGFR, ADRB1, ADRA2A, AVPR2, ADRA2C, EDNRA |
| chronic disease | ADORA3, ADORA2B, ADGRG6, ADRB2, CASR, GABBR1, AGTR1, GABBR2, ADORA1, CHRM3, CHRM1, ADORA2A |
| connective tissue disease | ADRB3, OPRD1, MC2R, OPRK1, CASR, PTGER3, OPRM1, ADRA2A, ADRA2B, ADRA2C, GNRHR |
| digestive system disease | CALCR, AGTR1, MTNR1B, GIPR, CHRM3, SSTR3, SSTR1, HTR4, PTGER3, GCGR, CHRM1, EDNRB, HRH2, DRD3, OPRM1, GLP1R, P2RY12, S1PR1, SSTR2, HTR1A, DRD4, MC1R, EDNRA, GLP2R, SSTR5 |
| disorder of development or morphogenesis | CALCR, S1PR4, AGTR1, GABBR2, CHRM3, GNRHR, KISS1R, S1PR3, FZD2, PTH1R, HRH1, S1PR2, EDNRB, LHCGR, ADGRG2, ADGRG6, CELSR1, GABBR1, SMO, S1PR1, MC2R, S1PR5, MC1R, EDNRA |
| disorder of visual system | ADRB3, ADRB2, GPR179, GPR143, P2RY2, OPN1LW, CHRM3, P2RY4, OPN1SW, GRM6, CASR, HRH1, CHRM1, FZD4, EDNRB, RHO, ADRA2B, ADGRV1, FZD5, CHRM2, P2RY1, PTGFR, ADRB1, ADRA2A, ADRA2C, P2RY6, OPN1MW |
| endocrine system disease | CALCR, AGTR1, MTNR1B, MC4R, GIPR, TSHR, SSTR3, SSTR1, TACR3, GNRHR, FSHR, KISS1R, PTH1R, CASR, GCGR, GHRHR, LHCGR, DRD3, SSTR4, GHSR, GLP1R, TACR1, P2RY12, MC2R, SSTR2, PROKR2, DRD4, MC1R, EDNRA, CHRM1, SSTR5 |
| genetic disorder | CNR1, GABBR2, MC4R, SSTR3, SSTR1, KISS1R, PTH1R, GHRHR, ADRA2B, GRM1, AVPR1A, P2RY12, HTR1A, PROKR2, OPN1MW, TBXA2R, ADRA1A, AGTR1, MTNR1B, OPN1LW, CHRM3, GRM7, HTR1D, GPR68, S1PR2, RHO, LHCGR, ADORA2A, ADORA3, ADORA2B, SSTR4, BDKRB2, LPAR6, ADGRV1, S1PR1, SSTR2, CHRM2, PTGFR, HRH3, CALCR, HTR2A, GPR143, MTNR1A, TACR3, HTR4, GNRHR, FSHR, GRM6, FZD2, CASR, EDNRB, ADRA1D, ADGRG2, ADGRG6, GABBR1, ADRA1B, OPRM1, SMO, FZD6, MC2R, ADRA2A, AVPR2, ADRA2C, CHRM1, SSTR5, ADRB3, GPR179, ADORA1, TSHR, OPN1SW, DRD2, CXCR4, FZD4, AVPR1B, HRH2, DRD3, CELSR1, CCR5, HTR2C, DRD4, MC1R, EDNRA |
| head and neck disorder | ADRB3, ADRB2, GPR179, ADRA1A, GPR143, P2RY2, OPN1LW, CHRM3, P2RY4, OPN1SW, GRM6, CASR, HRH1, FZD4, EDNRB, ADRA1D, RHO, OPN1MW, ADRA1B, ADRA2B, ADGRV1, FZD5, CHRM2, P2RY1, PTGFR, ADRB1, ADRA2A, ADRA2C, P2RY6, CHRM1 |
| hematologic disease | P2RY12, MC2R, CXCR4, TBXA2R, OPRM1, DRD4, AVPR2, AVPR1B, AVPR1A, SMO, DRD3 |
| idiopathic disease | HTR2A, CASR, HRH1, CNR1, GABBR2, EDNRB, EDNRA, HTR2C |
| immune system disease | ADRB3, CALCR, ADRA1A, S1PR4, GABBR2, TSHR, CHRM3, S1PR3, CXCR4, HRH1, S1PR2, GCGR, ADRA1D, CYSLTR1, CELSR1, GABBR1, ADRA1B, ADRA2B, GLP1R, CCR5, S1PR1, MC2R, CHRM2, S1PR5, PTGFR, ADRA2A, ADRA2C, CHRM1 |
| inflammatory disease | ADRB3, OPRD1, ADRB2, ADRA1A, AGTR1, P2RY2, TSHR, CHRM3, P2RY4, HRH1, PTGER3, ADRA1D, CYSLTR1, ADRA1B, BDKRB2, OPRM1, ADRA2B, S1PR1, MC2R, OPRK1, CHRM2, P2RY1, ADGRE2, PTGFR, ADRB1, ADRA2A, ADRA2C, P2RY6, CHRM1 |
| injury | PTH1R, HRH1, GABBR1, PTGER3, GABBR2, AVPR2, AVPR1B, AVPR1A, HRH2, CHRM1 |
| integumentary system disease | ADRB3, HRH1, ADRA1A, GNRHR, ADRA1B, BDKRB2, ADGRE2, LPAR6, EDNRA, ADRA2A, ADRA2B, EDNRB, MC1R, PTGFR, ADRA1D, ADRA2C, SMO, FZD6 |
| metabolic disease | ADRB3, OPRD1, CALCR, HTR2A, OXTR, CNR1, GPR143, AGTR1, GABBR2, MC4R, MTNR1A, GIPR, CHRM3, TSHR, MTNR1B, SSTR3, SSTR1, DRD2, CASR, GCGR, DRD3, ADORA2A, SSTR4, OPRM1, GRM1, GLP1R, HTR2C, P2RY12, OPRK1, SSTR2, CHRM2, DRD4, ADRA2A, MC1R, EDNRA, CHRM1, SSTR5 |
| musculoskeletal system disease | ADRB3, OPRD1, ADRB2, CALCR, HTR2A, ADRA1A, AGTR1, GABBR2, FZD2, PTH1R, CASR, PTGER3, EDNRB, ADRA1D, GABBR1, ADRA1B, OPRM1, ADRA2B, SMO, HTR2C, MC2R, OPRK1, ADRB1, ADRA2A, ADRA2C, EDNRA |
| nervous system disease | OPRD1,HCRTR1,DRD1,GRM3,CNR1,GABBR2,SSTR3,ADGRG1,S STR1,GHRHR,HTR1F,HTR7,ADRA2B,GRM1,HCRTR2,P2RY12,HT R1A,S1PR5,OPN1MW,ADRB2,HTR2B,ADRA1A,AGTR1,MTNR1B, OPN1LW,HTR6,CHRM3,GRM7,HTR1D,S1PR3,S1PR2,RHO,ADOR A2A,ADORA3,ADORA2B,SSTR4,DRD5,CALCRL,ADGRV1,HTR1 E,S1PR1,SSTR2,CHRM2,HTR5A,HRH3,HTR2A,OXTR,GPR143,MT NR1A,HTR4,GRM6,CASR,HRH1,HTR1B,ADRA1D,GABBR1,ADR A1B,OPRM1,SMO,MC2R,OPRK1,ADRA2A,ADRA2C,CHRM1,SST R5,GPR179,S1PR4,ADORA1,0PN1SW,DRD2,FZD4,DRD3,CYSLT R1,CELSR1,HTR2C,DRD4,ADRB1 |
| obstetric disorder | ADRB3, OXTR, ADRA1A, PTGER3, ADRA1B, TSHR, ADRA2A, ADRA2B, GLP1R, ADRA1D, ADRA2C |
| otorhinolaryngol ogic disease | ADRB3, ADRB2, CYSLTR1, HRH1, ADRA1A, CHRM2, ADRA1B, CHRM3, ADRA2A, ADRA2B, ADRB1, ADRA1D, ADRA2C, EDNRA, CHRM1 |
| psychiatric disorder | OPRD1, HCRTR1, HTR2A, DRD1, OXTR, HTR2B, ADRA1A, HRH3, GRM3, GABBR2, MTNR1A, HTR6, MTNR1B, HTR1D, HTR4, DRD2, HRH1, HTR1B, AVPR1B, DRD3, HTR1F, CYSLTR1, DRD5, GABBR1, OPRM1, HTR7, ADRA2B, GRM1, HCRTR2, AVPR1A, HTR2C, HTR1E, OPRK1, HTR1A, DRD4, ADRA2A, AVPR2, HTR5A, ADRA2C, CHRM1 |
| reproductive system disease | ADRA1A,SSTR3,SSTR1,TACR3,GNRHR,FSHR,KISS1R,ADRA1D, LHCGR,ADGRG2,DRD3,SSTR4,PTGER2,ADRA1B,PTGER1,CCR5, TACR1,SSTR2,PROKR2,DRD4,AVPR2,SSTR5 |
| respiratory system disease | ADRB3,ADRB2,ADRA1A,AGTR1,ADORA1,CHRM3,PTGIR,CASR ,HRH1,EDNRB,ADRA1D,ADORA2A,ADORA3,ADORA2B,ADGR G6,CYSLTR1,ADRA1B,ADRA2B,CHRM2,ADRB1,ADRA2A,ADR A2C,EDNRA,CHRM1 |
| syndromic disease | HTR2A,P2RY2,GABBR2,CHRM3,SSTR3,P2RY4,SSTR1,HTR4,GN RHR,FSHR,KISS1R,DRD2,FZD2,CASR,EDNRB,LHCGR,DRD3,CY SLTR1,SSTR4,GABBR1,OPRM1,ADGRV1,HTR2C,P2RY12,MC2R, SSTR2,HTR1A,PROKR2,P2RY1,DRD4,P2RY6,CHRM1,SSTR5 |
| upper digestive tract disorder | HTR4, HRH2 |
| urinary system disease | ADRB3, ADRA1A, AGTR1, TSHR, CHRM3, CASR, EDNRB, AVPR1B, ADRA1D, ADORA2A, ADORA2B, ADRA1B, OPRM1, ADRA2B, AVPR1A, MC2R, CHRM2, ADRA2A, AVPR2, ADRA2C, EDNRA |

### Agents that Modulate Target Proteins

Provided herein are agents that modulate the expression of a target protein disclosed herein, such as a target protein in the Sequence Listing, Table A, or a variant of the foregoing, or a fragment of the foregoing (e.g., a biologically active fragment of a target protein). The expression of the target protein or variant or fragment thereof can be modulated by a wide range of processes, directly or indirectly, leading to an increase or a decrease of the target protein level. Non-limiting examples include altering: the copy number of the gene encoding the target protein, transcriptional initiation, elongation or termination, RNA processing, RNA stability (e.g., mRNA stability), RNA degradation, translation initiation, post-translational modification of a protein, protein stability, protein degradation (e.g., cleavage, such as protease cleavage), or a combination of the foregoing.

In some embodiments, the agent modulates (e.g., increases or decreases) the expression of a gene or gene transcript encoding the target protein. In some embodiments, the agent modulates the expression or activity of the target protein. In some embodiments, the agent decreases (e.g., inhibits, reduces or neutralizes) the activity of the target protein. In some embodiments, the agent increases (e.g., activates) the activity of the target protein. In some embodiments, the agent decreases (e.g., inhibits or downregulates) the expression of the target protein. In other embodiments, the agent increases (e.g., activates or upregulates) the expression of the target protein.

As used herein, the term "increasing" or "increase" refers to modulation that results in a higher level of expression, activity, function or a combination thereof of the target protein, or a metric (e.g., cancer cell death or DNA methylation of a target site), relative to a reference (e.g., the level prior to or in an absence of modulation by the agent). In some embodiments, the agent increases the expression or activity of the target protein, or the metric, by at least about 5% relative to the reference, e.g., by at least about: 10%, 15%, 20%, 25%, 30%, 35%, 40%, 45%, 50%, 55%, 60%, 65%, 70%, 75%, 80%, 85%, 90%, 95% or 98% relative to the reference.

As used herein, the term "decreasing" or "decrease" refers to modulation that results in a lower level of expression, activity, function or a combination thereof of the target protein, or a metric (e.g., cancer cell death or DNA methylation of a target site), relative to a reference (e.g., the level prior to or in an absence of modulation by the agent). In some embodiments, the agent decreases the expression or activity of the target protein, or the metric, by at least about 5% relative to the reference, e.g., by at least about: 10%, 15%, 20%, 25%, 30%, 35%, 40%, 45%, 50%, 55%, 60%, 65%, 70%, 75%, 80%, 85%, 90%, 95% or 98% relative to the reference.

Non-limiting examples of the metric include energy production or energy conversion in the liver (e.g., modulating ATP synthesis, B-oxidation, oxidation of metabolites derived from glycolysis, oxidation of metabolites derived from amino acids), mitochondrial transcription, mitochondrial ribosome assembly, mitochondrial translation, mitochondrial thermogenesis, hormonal signaling (e.g., mitochondrial estrogen receptor (mtER) signaling), redox maintenance (e.g., NADH and/or FADH₂), cell cycle regulation, cell migration, cell morphology, apoptosis, necrosis, membrane potential, ion (e.g., calcium or zinc) storage, ion (e.g., calcium or zinc) homeostasis, metabolite synthesis (e.g., heme biosynthesis or steroid biosynthesis), nutrient sensing, unfolded protein stress response pathway, signaling processes (e.g., calcium signaling).

In some embodiments, the level of expression, activity, function or a combination thereof of the target protein, or the metric, is measured after the agent is contacted with (e.g., a cell) or administered (e.g., to a subject) for at least about 1 day, e.g., at least about: 2 days, 3 days, 4 days, 5 days, 6 days, 8 days, 9 days, 10 days, 1 week, 2 weeks, 3 weeks, 1 month, 2 months, 3 months, 4 months, 5 months or 6 months, e.g., after a treatment regimen has begun.

In some embodiments, the agent comprises, consists essentially of or consists of a polypeptide, a polynucleotide, a gene editing system, a small molecule, or a cell (e.g., a cell therapy).

In some embodiments, the target protein is a tumor suppressor, and the agent increases the level of expression, activity, function (e.g., tumor suppressor function) or a combination thereof, of the target protein. In other embodiments, the target protein is an oncogene, and the agent decreases the level of expression, activity (e.g., oncogenic activity), function or a combination thereof of the target protein.

In certain embodiments, the agent modulates the level of expression, activity, function, or a combination thereof, of the target protein in a cancer cell (e.g., a metastatic cancer cell), a cell (e.g., stromal cell) in a tumor micro-environment, a non-malignant cell, or a combination of the foregoing. In certain embodiments, the agent modulates the level of expression, activity, function or a combination thereof of the target protein in a tumor, a tumor microenvironment, a site of metastasis, a stromal cell, or a combination of the foregoing.

In some embodiments, the agent induces downregulation of the target protein (e.g., increases target-protein degradation); prevents multimerization (e.g., dimerization) of the target protein; sequesters a target protein (e.g., a secreted target protein); modulates (e.g., agonizes, antagonizes or disrupts) a known function of the target protein; decreases binding between the target protein and a binding partner (e.g., via steric hinderance); induces antibody-dependent cell killing, phagocytosis, and/or opsonization of a cell expressing the target protein; or a combination of the foregoing. In certain embodiments, the agent lacks agonistic activity toward the target protein. In certain embodiments, the agent has agonistic activity toward the target protein. In some embodiments, the agent lacks antagonistic activity toward the target protein. In some embodiments, the agent has antagonistic activity toward the target protein. In particular embodiments, the agent binds to at least one residue of the target protein that is involved in binding to a binding partner. In some embodiments, the agent binds to one or more binding sites and/or domains of the target protein involved in binding of the target protein to the binding partner.

In some embodiments, the agent induces downregulation of a binding partner of the target protein; sequesters a binding partner (e.g., a secreted binding partner) of the target protein; prevents multimerization (e.g., dimerization) of a binding partner of the target protein; sequesters a binding partner of the target protein (e.g., a secreted binding partner); modulates (e.g., agonizes, antagonizes or disrupts) a known function of a binding partner of the target protein; decreases binding between the target protein and a binding partner (e.g., via steric hinderance); induces antibody-dependent cell killing, phagocytosis, and/or opsonization of a cell expressing a binding partner of the target protein; or a combination of the foregoing. In certain embodiments, the agent lacks agonistic activity toward a binding partner of the target protein. In certain embodiments, the agent has agonistic activity toward a binding partner of the target protein. In some embodiments, the agent lacks antagonistic activity toward a binding partner of the target protein. In some embodiments, the agent has antagonistic activity toward a binding partner of the target protein. In particular embodiments, the agent further binds to at least one residue of a binding partner of the target protein that is involved in binding between the target protein and the binding partner. In more particular embodiments, the agent further binds to one or more binding sites and/or domains of a binding partner of the target protein involved in binding between the target protein and the binding partner.

In certain embodiments, the agent modulates (e.g., increases or decreases) the level of expression, activity, function, or a combination thereof, of a variant of a target protein disclosed herein. In some embodiments, the variant comprises an amino acid sequence that is at least 70% identical to the amino acid sequence of a target protein disclosed herein. For example, the sequence identity to the variant can be at least about: 75%, 80%, 85%, 90%, 91%, 92%, 93%, 94%, 95%, 96%, 97%, 98% or 99%. In some embodiments, the sequence identity is about: 70%, 75%, 80%, 85%, 90%, 91%, 92%, 93%, 94%, 95%, 96%, 97%, 98% or 99%. In some embodiments, the sequence identity is about: 70-99%, 75-99%, 75-95%, 80-99%, 80-98%, 80-95%, 80-90%, 85-98%, 85-97%, 85-90%, 90-97%, 90-96%, 90-85%, 90-80% or 95-99%. In some embodiments, a variant comprises an amino acid sequence that is at least about 75%, at least about 80%, at least about 85%, at least about 90%, at least about 95%, or at least about 98% identical to the amino acid sequence of a target protein disclosed herein.

As used herein, the term "sequence identity," refers to the extent to which two nucleotide sequences, or two amino acid sequences, have the same residues at the same positions when the sequences are aligned to achieve a maximal level of identity, expressed as a percentage. For sequence alignment and comparison, typically one sequence is designated as a reference sequence, to which a test sequences are compared. The sequence identity between reference and test sequences is expressed as the percentage of positions across the entire length of the reference sequence where the reference and test sequences share the same nucleotide or amino acid upon alignment of the reference and test sequences to achieve a maximal level of identity. As an example, two sequences are considered to have 70% sequence identity when, upon alignment to achieve a maximal level of identity, the test sequence has the same nucleotide or amino acid residue at 70% of the same positions over the entire length of the reference sequence.

Alignment of sequences for comparison to achieve maximal levels of identity can be readily performed by a person of ordinary skill in the art using an appropriate alignment method or algorithm. In some instances, the alignment can include introduced gaps to provide for the maximal level of identity. Examples include the local homology algorithm of Smith & Waterman, Adv. Appl. Math. 2:482 (1981), the homology alignment algorithm of Needleman & Wunsch, J. Mol. Biol. 48:443 (1970), the search for similarity method of Pearson & Lipman, Proc. Nat'l. Acad. Sci. USA 85:2444 (1988), computerized implementations of these algorithms (GAP, BESTFIT, FASTA, and TFASTA in the Wisconsin Genetics Software Package, Genetics Computer Group, 575 Science Dr., Madison, Wis.), and visual inspection (see generally Ausubel et al., Current Protocols in Molecular Biology).

When using a sequence comparison algorithm, test and reference sequences are input into a computer, subsequent coordinates are designated, if necessary, and sequence algorithm program parameters are designated. The sequence comparison algorithm then calculates the percent sequence identity for the test sequence(s) relative to the reference sequence, based on the designated program parameters. A commonly used tool for determining percent sequence identity is Protein Basic Local Alignment Search Tool (BLASTP) available through National Center for Biotechnology Information, National Library of Medicine, of the United States National Institutes of Health. (Altschul *et al.,* 1990).

In some embodiments, the amino acid sequence of a variant of a target polypeptide disclosed herein comprises at least one amino acid substitution relative to the amino acid sequence of the target protein. In some embodiments, the number of amino acid substitutions in a variant relative to the amino acid sequence of a target protein disclosed herein is at least about: 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60. In some embodiments, the number of amino acid substitutions is at least about: 5, 6, 10, 15, 20, 25, 30, 35, 40, 45, 50, 55 or 60. In some embodiments, the number of amino acid substitutions is up to about: 60, 55, 50, 45, 40, 35, 30, 25, 20, 15, 10, 6 or 5. In some embodiments, the number of amino acid substitutions is about: 1-60, 1-55, 2-55, 2-50, 3-50, 3-45, 4-45, 4-40, 5-40, 5-35, 6-35, 6-30, 7-30, 7-25, 8-25, 8-20, 9-20, 9-15, 10-15, 5-60, 10-60, 10-55, 15-55, 15-50, 20-50, 20-45, 25-45, 25-40 or 30-40. In some embodiments, the number of amino acid substitutions is about: 10-35, 10-33, 11-33, 11-31, 12-31, 12-29, 13-29, 13-27, 14-27 or 14-25.

The amino acid substitution(s) in a variant can be substitutions with a canonical amino acid or a non-canonical amino acid. Non-canonical amino acids include, but are not limited to D amino acids, such as D versions of the canonical L-amino acids.

In some embodiments, an amino acid substitution is a conservative substitution. The term "conservative amino acid substitution(s)" or "conservative substitution(s)" refers to an amino acid substitution having a value of 0 or greater in BLOSUM62.

In some embodiments, an amino acid substitution is a highly conservative substitution. The term "highly conservative amino acid substitution(s)" or "highly conservative substitution(s)" refers to an amino acid substitution having a value of at least 1 (*e.g.,* at least 2) in BLOSUM62.

In some embodiments, a variant of a target protein of the disclosure comprises about 5-60 amino acid substitutions, relative to the amino acid sequence of a target protein disclosed herein. In some embodiments, the amino acid substitutions include at least one conservative substitution. In some embodiments, the amino acid substitutions include at least one highly conservative substitution.

### A. Polypeptide Agents

The term "polypeptide" "peptide" or "protein" denotes a polymer of at least two amino acids covalently linked by an amide bond, regardless of length or post-translational modification (*e.g*., glycosylation or phosphorylation). A protein, peptide or polypeptide can comprise any suitable L-and/or D-amino acid, for example, common α-amino acids (*e.g.,* alanine, glycine, valine), non-α-amino acids (*e.g*., β-alanine, 4-aminobutyric acid, 6-aminocaproic acid, sarcosine, statine), and unusual amino acids (*e.g*., citrulline, homocitruline, homoserine, norleucine, norvaline, ornithine). The amino, carboxyl and/or other functional groups on a peptide can be free (*e.g*., unmodified) or protected with a suitable protecting group. Suitable protecting groups for amino and carboxyl groups, and methods for adding or removing protecting groups are known in the art and are disclosed in, for example, Green and Wuts, "Protecting Groups in Organic Synthesis, " John Wiley and Sons, 1991. The functional groups of a protein, peptide or polypeptide can also be derivatized (*e.g.,* alkylated) or labeled (*e.g.,* with a detectable label, such as a fluorogen or a hapten) using methods known in the art. A protein, peptide or polypeptide can comprise one or more modifications (*e.g*., amino acid linkers, acylation, acetylation, amidation, methylation, terminal modifiers (*e.g.,* cyclizing modifications), N-methyl-α-amino group substitution), if desired. In addition, a protein, peptide or polypeptide can be an analog of a known and/or naturally-occurring peptide, for example, a peptide analog having conservative amino acid residue substitution(s).

In some embodiments, the agent comprises a polypeptide. In some embodiments, the polypeptide is an isolated polypeptide (e.g., isolated or extracted from a biological sample or source). In some embodiments, the polypeptide is a recombinant polypeptide. In some embodiments, the polypeptide is an inhibitor (e.g., a direct inhibitor or an indirect inhibitor) of the expression and/or activity of a target protein disclosed herein. In some embodiments, the polypeptide is an activator (e.g., a direct activator or an indirect activator) of the expression and/or activity of a target protein disclosed herein. In some embodiments, the polypeptide decreases the expression or activity of the target protein disclosed herein. In other embodiments, the polypeptide increases the expression or activity of the target protein disclosed herein. In some embodiments, the polypeptide is a target protein disclosed herein, or a portion thereof (e.g., a biologically active portion thereof, such as a biologically active fragment of the target protein).

In some embodiments, the polypeptide is an immunoglobulin molecule, such as an antibody (e.g., a whole antibody, an intact antibody) or an antigen-binding fragment of an antibody. In some embodiments, the antibody or antigen-binding fragment thereof binds to the target protein. In some embodiments, the antibody or antigen-binding fragment thereof binds to a protein capable of modulating the expression or activity of the target protein.

In some embodiments, the polypeptide is an antibody. As used herein, the term "antibody" refers to an immunoglobulin molecule capable of specific binding to a target, such as a carbohydrate, polynucleotide, lipid, polypeptide, etc., through at least one antigen recognition site, located in the variable region of the immunoglobulin molecule. As used herein, the term "antibody" refers to a full-length antibody comprising two heavy (H) chains and two light (L) chains inter-connected by disulfide bonds or multimers thereof (for example, IgM). Each heavy chain comprises a heavy chain variable region (V_{H}) and a heavy chain constant region (comprising domains CH1, hinge CH2 and CH3). Each light chain comprises a light chain variable region (V_{L}) and a light chain constant region (CL). The V_{H} and the V_{L} regions may be further subdivided into regions of hypervariability, termed complementarity determining regions (CDR), interspersed within framework regions (FR). V_{H} and V_{L} each comprises three CDRs and four FR segments, arranged from the amino-terminus to the carboxy-terminus in the following order: FR1, CDR1, FR2, CDR2, FR3, CDR3, and FR4. The antibody can be of any species, such as a rodent (e.g., murine, rat, guinea pig) antibody, a human antibody, or the antibody can be a humanized antibody or chimeric antibody.

In some embodiments, the antibody comprises an IgA (e.g., IgA1 or IgA2) heavy chain constant region, an IgD heavy chain constant region, an IgE heavy chain constant region, an IgG (e.g., IgG1, IgG2 (e.g., IgG2a, IgG2b or IgG2c), IgG3 or IgG4) heavy chain constant region or an IgM heavy chain constant region. In some embodiments, the antibody comprises an IgG heavy chain constant region. In some embodiments, the antibody comprises a κ light chain constant region. In some embodiments, the antibody comprises a λ light chain constant region.

In some embodiments, the antibody is a polyclonal antibody. In some embodiments, the antibody is a monoclonal antibody. In some embodiments, the antibody is human or chimeric. In some embodiments, the antibody is primatized (e.g., humanized). In some embodiments, the antibody is multispecific, e.g., bi-, tri-, or quad-specific. In some embodiments, the antibody is a heteroconjugate antibody.

In some embodiments, the polypeptide agent is an antigen-binding fragment of an immunoglobulin molecule (e.g., antibody). The term "antigen-binding fragment" refers to a portion of an immunoglobulin molecule (e.g., antibody) that retains the antigen binding properties of the parental full-length antibody. Non-limiting examples of antigen-binding fragments include a V_{H} region, a V_{L} region, an Fab fragment, an F(ab')₂ fragment, an Fd fragment, an Fv fragment, and a domain antibody (dAb) consisting of one V_{H} domain or one V_{L} domain, etc. V_{H} and V_{L} domains may be linked together via a synthetic linker to form various types of single-chain antibody designs in which the V_{H}/V_{L} domains pair intramolecularly, or intermolecularly in those cases when the V_{H} and V_{L} domains are expressed by separate chains, to form a monovalent antigen binding site, such as single chain Fv (scFv) or diabody. In some embodiments, the polypeptide disclosed herein is an antigen binding fragment selected from Fab, Fab', F(ab')₂, Fd, Fv, disulfide-linked Fvs (sdFv, e.g., diabody, triabody or tetrabody), scFv, SMIP or rlgG. In some embodiments, the polypeptide is a scFv. Antigen-binding fragments can be produced by recombinant DNA techniques, enzymatic or chemical cleavage of intact immunoglobulins, or, in certain cases, by chemical peptide synthesis procedures known in the art.

Polypeptide agents (e.g., monoclonal antibodies) can be monovalent, bivalent or multivalent. A monoclonal antibody can be monospecific or multispecific (e.g., bispecific). Monospecific antibodies bind one antigenic epitope. A multispecific antibody, such as a bispecific antibody or a trispecific antibody, is included in the term monoclonal antibody.

"Multispecific" refers to an antibody that specifically binds at least two distinct antigens or at least two distinct epitopes within the antigens, for example three, four or five distinct antigens or epitopes. "Bispecific" refers to an antibody that specifically binds two distinct antigens or two distinct epitopes within the same antigen.

"Isolated antibody" refers to an antibody or an antigen-binding fragment thereof that is substantially free of other antibodies having different antigenic specificities (e.g., an isolated anti-target protein antibody is substantially free of antibodies that specifically bind antigens other than the target protein). In the case of a bispecific antibody, the bispecific antibody specifically binds two antigens of interest, and is substantially free of antibodies that specifically bind antigens other than the two antigens of interest. In some embodiments, the polypeptide agent (e.g., monoclonal antibody) is at least 80% pure, *e.g.,* about: 80%, 81%, 82%, 83%, 84%, 85%, 86%, 87%, 88%, 89%, 90%, 91%, 92%, 93%, 94%, 95%, 96%, 97%, 98%, 99% or 100% pure.

In some embodiments, the polypeptide is an antagonist antibody that binds to the target protein (e.g., a target protein whose expression or activity is increased in a cancer state relative to a reference state). In some embodiments, the antibody described herein is an antagonist antibody that binds to a protein capable of modulating the expression or activity of the target protein. As used herein, the term "antagonist antibody" refers to an antibody that, upon binding to an antigen (e.g., the target protein or a protein capable of modulating the expression or activity of the target protein), reduces (e.g., inhibits) the function of the antigen. In some embodiments, the antigen is a receptor, and the antagonist antibody binds to the ligand-binding domain of the receptor. In some embodiments, the antigen is a transmembrane protein, and the antagonist antibody binds to the extracellular region of the transmembrane protein. In some embodiments, the antigen is an enzyme or a signaling molecule, and the antagonist antibody reduces the activity of the enzyme or attenuate a signal transduction pathway mediated by the signaling molecule. In some embodiments, the antagonist antibody reduces antigen function by at least about 10%, e.g., by at least about: 15%, 20%, 25%, 30%, 35%, 40%, 45%, 50%, 55%, 60%, 65%, 70%, 75%, 80%, 90%, 95%, 98% or 99%.

In some embodiments, the polypeptide is an agonist antibody that binds to the target protein (e.g., a target protein whose expression or activity is decreased in a cancer state relative to a reference state). In some embodiments, the antibody is an agonist antibody that binds to a protein capable of modulating the expression or activity of the target protein. As used herein, the term "agonist antibody" refers to an antibody that, upon binding to an antigen (e.g., the target protein or a protein capable of modulating the expression or activity of the target protein), increases the function of the antigen. In some embodiments, the antigen is a receptor, and the agonist antibody binds to the ligand-binding domain of the receptor. In some embodiments, the antigen is a transmembrane protein, and the agonist antibody binds to the extracellular region of the transmembrane protein. In some embodiments, the antigen is an enzyme or a signaling molecule, and the agonist antibody increases the activity of the enzyme or activates a signal transduction pathway mediated by the signaling molecule. In some embodiments, the agonist antibody increases antigen function by at least about 10%, e.g., by at least about: 15%, 20%, 25%, 30%, 35%, 40%, 45%, 50%, 55%, 60%, 65%, 70%, 75%, 80%, 90%, 95%, 100%, 200%, 300%, 400%, 500%, 600%, 700%, 800%, 900% or 1,000%.

In some embodiments, the agonist antibody does not exert at least one of the following functional properties: reducing (e.g., inhibiting) the activity of the antigen; inducing antibody-dependent cell killing of a cell expressing the antigen (e.g., by natural killer (NK) cells, monocytes, macrophages, neutrophils, dendritic cells, or eosinophils); inducing phagocytosis of a cell expressing the antigen (e.g., by macrophage); inducing opsonization of a cell expressing the antigen; and inducing downregulation of the antigen on a cell surface (e.g., by hyper-crosslinking or clustering the antigen to induce internalization and degradation).

Suitable techniques, assays and reagents for making and using therapeutic antibodies against an antigen are known in the art. *See,* for example, Therapeutic Monoclonal Antibodies: From Bench to Clinic (Zhiqiang An eds., 1st ed. 2009); Antibodies: A Laboratory Manual (Edward A. Greenfield eds., 2d ed. 2013); Ferrara et al., Using Phage and Yeast Display to Select Hundreds of Monoclonal Antibodies: Application to Antigen 85, a Tuberculosis Biomarker, PLoS ONE 7(11): e49535 (2012), for methods of making recombinant antibodies, including antibody engineering, use of degenerate oligonucleotides, 5'-RACE, phage display, and mutagenesis; antibody testing and characterization; antibody pharmacokinetics and pharmacodynamics; antibody purification and storage; and screening and labeling techniques.

In some embodiments, the polypeptide is an antibody mimetic that binds a target protein disclosed herein. The term "antibody mimetic" refers to polypeptides capable of mimicking an antibody's ability to bind an antigen, but structurally differ from native antibody structures. Non-limiting examples of antibody mimetics include Adnectins, Affibodies, Affilins, Affimers, Affitins, Alphabodies, Anticalins, Avimers, DARPins, Fynomers, Kunitz domain peptides, monobodies, nanobodies, nanoCLAMPs, and Versabodies.

In some embodiments (e.g., when the expression or activity of the target protein is decreased in a cancer state relative to a reference state), the agent is a polypeptide (e.g., isolated polypeptide) that comprises an amino acid sequence that is at least 70% identical to at least a portion (e.g., a biologically active portion or fragment) of the target protein. For example, the percent identity can be at least about: 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% to the full-length target protein or a biologically active portion or fragment thereof. In some embodiments, the polypeptide comprises the amino acid sequence of a full-length target protein. In some embodiments, the polypeptide comprising the amino acid sequence of a full-length target protein is a recombinant polypeptide. In some embodiments, the polypeptide comprising the amino acid sequence of a full-length target protein is a synthetic polypeptide.

In some embodiments, the polypeptide (e.g., isolated polypeptide) comprises an amino acid sequence having at least 1 amino acid substitution relative to the target protein. For example, the number of amino acid substitutions can be at least about: 2, 3, 4, 5, 6, 7, 8, 9, 10, 11, 12, 13, 14, 15, 16, 17, 18, 19 or 20, or about: 1-20, 1-19, 2-19, 2-18, 2-17, 3-17, 3-16, 4-16, 4-15, 5-15, 5-14, 6-14, 6-13, 7-13, 7-12, 8-12, 8-11 or 9-11. In some embodiments, the amino acid substitutions are conservative substitutions. In some embodiments, the amino acid substitutions are highly conservative substitutions.

In some embodiments, the polypeptide (e.g., isolated polypeptide) comprises an amino acid sequence that is at least 70% identical to at least a portion of a protein capable of modulating the expression or activity of the target protein. For example, the percent identity can be at least about: 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%. In some embodiments, the polypeptide comprises the amino acid sequence of a protein capable of modulating the expression or activity of the target protein.

In some embodiments, the polypeptide (e.g., isolated polypeptide) comprises an amino acid sequence having at least 1 amino acid substitution relative to a protein capable of modulating the expression or activity of the target protein. For example, the number of amino acid substitutions can be at least about: 2, 3, 4, 5, 6, 7, 8, 9, 10, 11, 12, 13, 14, 15, 16, 17, 18, 19 or 20, or about: 1-20, 1-19, 2-19, 2-18, 2-17, 3-17, 3-16, 4-16, 4-15, 5-15, 5-14, 6-14, 6-13, 7-13, 7-12, 8-12, 8-11 or 9-11. In some embodiments, the amino acid substitutions are conservative substitutions. In some embodiments, the amino acid substitutions are highly conservative substitutions.

In some embodiments, the polypeptide is a cell-penetrating peptide. In certain embodiments, the polypeptide is linked to a cell-penetrating peptide. Suitable cell-penetrating peptide sequences can be protein-derived, designed or, chimeric (modified). *See,* e.g., Regberg, et al., Applications of cell-penetrating peptides for tumor targeting and future cancer therapies, Pharmaceuticals 5(9): 991-1007 (2012). Non-limiting examples of cell-penetrating peptides include TAT (48-60), Penetratin, pVEC, MPG8, Transportan, Transportan10, PepFect3, PepFect 6, PepFect 14, Polyarginine, Stearyl-polyarginine, Pep-1, Pep-3, CADY, YTA2, YTA4, SynB1, SynB3, Maurocalcine and PTD4.

In some embodiments, the polypeptide is a circulating factor (e.g., a cytokine).

In some embodiments, the biological properties (e.g., biological activities or half-life) of the polypeptide (e.g., isolated polypeptide) and the target protein are similar. Non-limiting examples of biological activities include enzymatic activities or properties (e.g., selectivity, steady-state or kinetics), binding activities (e.g., nucleic acid (DNA, RNA) binding protein binding) or properties (e.g., specificity, affinity or kinetics), cell signaling activities, immunological activities, and structural activity (e.g., cell adhesion), among others. Non-limiting examples of enzyme activities include transferase activity (e.g., transfers functional groups from one molecule to another), oxioreductase activity (e.g., catalyzes oxidation-reduction reactions), hydrolase activity (e.g., cleaves chemical bonds via hydrolysis), lyase activity (e.g., generate a double bond), ligase activity (e.g., joining two molecules via a covalent bond), and isomerase activity (e.g., catalyzes structural changes within a molecule from one isomer to another).

In some embodiments, the polypeptide (e.g., isolated polypeptide) is a recombinant protein. In other embodiments, the polypeptide (e.g., isolated polypeptide) is a synthetic protein. Methods of producing therapeutic polypeptides are known in the art. *See,* e.g., Therapeutic Proteins: Methods and Protocols (Mark C. Smales & David C James eds., 2005*);* Pharmaceutical Biotechnology: Fundamentals and Applications (Daan J. A. Crommelin, Robert D. Sindelar & Bernd Meibohm eds., 2013). Polypeptides can be expressed recombinantly using mammalian cells, insect cells, yeast or bacteria *etc.,* under the control of appropriate promoters.

In some embodiments, the polypeptides described herein (e.g., target proteins, or a portion thereof, polypeptide agents that modulate target proteins) are modified, for example, by cleavage (e.g., protease cleavage) or post-translational modification. In certain embodiments, the modification(s) will affect the activity of the polypeptide, for example, by making an inactive polypeptide active or by altering (e.g., increasing, decreasing) the level of activity of a polypeptide. In particular embodiments, a polypeptide described herein is provided as a prodrug, e.g., that can be converted (e.g., by proteolytic cleavage, post-translational modification) to an active polypeptide *in vivo.* In some embodiments, the polypeptide includes a post-translational modification or other chemical modification. Non-limiting examples of post-translational modifications include acetylation, amidation, formylation, glycosylation, hydroxylation, methylation, myristoylation, phosphorylation, deamidation, prenylation (e.g., farnesylation, geranylation, etc.), ubiquitylation, ribosylation and sulphation. Phosphorylation can occur on an amino acid such as tyrosine, serine, threonine, or histidine.

In some embodiments, the polypeptide is joined to a heterologous peptide or protein (e.g., via a covalent bond such as a peptide bond, or a non-covalent bond), such as in a conjugate or fusion protein. In some embodiments, the polypeptide comprises a tag (e.g., a detectable label, such as a fluorophore or enzyme, or a purification tag, such as an epitope tag).

In some embodiments, the polypeptide comprises one or more neoantigens selected from the Sequence Listing, Table A, or a variant thereof. As used herein, the term "neoantigen" refers to a tumor antigen that arises from a target protein described herein. In some embodiments, the neoantigen is a cancer-specific neoantigen. There are a variety of ways to produce a neoantigen. For example, a neoantigen may be produced *in vitro* as a polypeptide before being formulated into a neoplasia vaccine or immunogenic pharmaceutical composition. In some embodiments, the immunogenic pharmaceutical composition comprises an effective amount of one or more neoantigens or pharmaceutically acceptable salt(s) thereof. In some embodiments, the immunogenic pharmaceutical composition further comprises a pharmaceutically acceptable carrier, excipient, adjuvant or additive.

Alternatively, a neoantigen may be produced *in vivo* by introducing a polynucleotide or an expression vector (e.g., a viral expression vector) encoding the neoantigen into a cell or tissue (e.g., of a subject in need). In certain embodiments, the polypeptide comprises at least two neoantigens. In some embodiments, the polypeptide comprises a T cell enhancer amino acid sequence. In some embodiments, the T cell enhancer is selected from the group consisting of an invariant chain, a leader sequence of tissue-type plasminogen activator, a PEST sequence, a cyclin destruction box, a ubiquitination signal, and a SUMOylation signal.

### B. Polynucleotide Agents

In some embodiments, the agent comprises a polynucleotide, or an analog or derivative thereof. In some embodiments, the polynucleotide, or the analog or derivative thereof, is an inhibitor of the target protein. In some embodiments, the polynucleotide, or the analog or derivative thereof, is an activator of the target protein. In some embodiments, the polynucleotide, or the analog or derivative thereof, decreases (e.g., reduces or neutralizes) the expression or activity of the target protein. In other embodiments, the polynucleotide, or the analog or derivative thereof, increases the expression or activity of the target protein.

The polynucleotides can have sequences containing naturally occurring ribonucleotide or deoxyribonucleotide monomers, non-naturally occurring nucleotides, or combinations thereof. Accordingly, polynucleotides can include, for example, nucleotides comprising naturally occurring bases (e.g., A, G, C, or T) and nucleotides comprising modified bases (e.g., 7-deazaguanosine, inosine, or methylated nucleotides, such as 5-methyl dCTP and 5-hydroxymethyl cytosine). In some embodiments, the polynucleotide comprises at least one modified nucleotide. Non-limiting examples of modified nucleotides include 2'-fluoro, 2'-o-methyl, 2'-deoxy, unlocked nucleic acid, 2'-hydroxy, phosphorothioate, 2'-thiouridine, 4'-thiouridine and 2'-deoxyuridine. In some embodiments, the modification increases nuclease resistance, increases serum stability, decrease immunogenicity, or a combination of the foregoing.

In some embodiments, the polynucleotide is a DNA molecule. In some embodiments, the polynucleotide is an RNA molecule. In some embodiments, the polynucleotide is a vector (e.g., expression vector, plasmid).

In some embodiments, the polynucleotide comprises an analog or a derivative of a polynucleotide. In some embodiments, the analog or derivative is a peptide nucleic acid (PNA). In some embodiments, the analog or derivative is a locked nucleic acid (LNA). In some embodiments, the analog or derivative is a morpholino oligonucleotide. In some embodiments, the analog or derivative comprises one or more phosphorothioate-linkages. In some embodiments, the agent comprises a deoxyribonucleic guanidine (DNG) nucleotide. In some embodiments, the agent comprises ribonucleic guanidine (RNG) nucleotide.

In some embodiments, the polynucleotide modulates the expression and/or activity of a nucleic acid encoding a target protein disclosed herein (e.g., a target protein in the Sequence Listing or Table A), a variant thereof, or a portion thereof (e.g., a biologically active portion or fragment thereof).

In some embodiments, the polynucleotide comprises a nucleotide sequence that is complementary (e.g., fully complementary or partially complementary) to at least a portion of a gene or gene transcript encoding a target protein disclosed herein, such that the polynucleotide sequence is capable of hybridizing or annealing to the gene or gene transcript (e.g., under physiological conditions). In other embodiments, the polynucleotide comprises a nucleotide sequence that is complementary to at least a portion of a gene or gene transcript encoding a protein that is capable of modulating the expression or activity of a target protein disclosed herein.

In some embodiments, the polynucleotide encodes a target protein disclosed herein, or a variant thereof (e.g., a biologically active variant thereof), or a portion thereof (e.g., a biologically active portion or fragment thereof).

In some embodiments, the nucleic acid that encodes the target protein, or variant thereof, or portion (e.g., fragment) thereof, is a gene sequence or a portion thereof. In some embodiments, the encoding nucleic acid is an unprocessed RNA transcript (e.g., pre-mRNA) or a portion thereof (e.g., a 5'-UTR, a 3'-UTR, an intron). In some embodiments the encoding nucleic acid is a mRNA molecule or a portion thereof. In some embodiments the encoding nucleic acid is present in a non-coding RNA (e.g., a long intergenic non-coding RNA (lincRNA), long noncoding RNA (lncRNA), or miRNA).

The encoding nucleic acid can comprise a canonical open reading frame (ORF) or a non-canonical ORF. In certain embodiments, the encoding nucleic acid comprises a non-canonical ORF.

The polynucleotides can be single stranded (ss) or double stranded (ds). In some embodiments, the polynucleotide is double stranded (ds). In some embodiments, the length of the ds polynucleotide is about 15-50 base pairs, e.g., about: 15-45, 15-40, 15-35, 15-30, 15-25, 18-50, 18-45, 18-40, 18-35, 18-30, 18-25, 20-50, 20-45, 20-40, 20-35, 20-30, 20-25, 25-50, 25-45, 25-40, 25-35, 25-30, 30-50, 30-45, 30-40, 30-35, 35-50, 35-45, 35-40 or 40-50 base pairs. In some embodiments, the length of the polynucleotide is about 19-23 base pairs. In some embodiments, the length of the polynucleotide is about 21 base pairs.

In some embodiments, the polynucleotide is single stranded (ss). In some embodiments, the length of the ss polynucleotide is about 15-50 nucleotides, e.g., about: 15-45, 15-40, 15-35, 15-30, 15-25, 18-50, 18-45, 18-40, 18-35, 18-30, 18-25, 20-50, 20-45, 20-40, 20-35, 20-30, 20-25, 25-50, 25-45, 25-40, 25-35, 25-30, 30-50, 30-45, 30-40, 30-35, 35-50, 35-45, 35-40 or 40-50 nucleotides.

In some embodiments, the polynucleotide prevents the maturation of a newly-generated nuclear RNA transcript into an mRNA for transcription. In some embodiments, the polynucleotide comprises a nucleotide sequence that is complementary to a sequence at the boundary of an intron and an exon.

In some embodiments, the polynucleotide (e.g., an antisense oligonucleotide) can hybridize to an mRNA encoding the target protein (e.g., under physiological conditions). In some embodiments, the length of the polynucleotide is at least about 10 nucleotides, e.g., at least about: 15, 16, 17, 18, 19, 20, 21, 22, 23, 24, 25, 26, 27, 28, 29 or 30 nucleotides or about: 10-30, 15-30, 15-25, 20-25 nucleotides. In some embodiments, the polynucleotide is at least 75% identical to an antisense sequence of identical the targeted transcript, e.g., at least about: 80%, 85%, 90%, 95%, 96%, 97%, 98% or 99%.

In some embodiments, the polynucleotide further comprises an overhang sequence (e.g., unpaired, overhanging nucleotides which are not directly involved in the formation of the double helical structure by the core sequences). In some embodiments, the polynucleotide comprises a 3' overhang, a 5' overhang, or both. In some embodiments, the overhang is about 1-5 nucleotides. In some embodiments, the overhang comprises a modified ribonucleotide or deoxynucleotide, e.g., a thiophosphate, phosphorothioate or deoxynucleotide inverted (3' to 3' linked) nucleotide.

Non-limiting examples of polynucleotide agents suitable for use in the compositions, kits and methods described herein include a small interfering RNA (siRNA), a short hairpin RNA (shRNA), a microRNA (miRNA), an antagomir, an antisense DNA, an antisense RNA, a morpholino nucleic acid (MNA), a locked nucleic acid (LNA), a peptide nucleic acid (PNA), an aptamer and a guide RNA (gRNA).

In some embodiments, the polynucleotide inhibits gene expression (e.g., through the biological process of RNA interference (RNAi)). Polynucleotides appropriate for RNA interference can be readily designed and produced by a person of ordinary skill using techniques, assays and reagents known in the art, including computational tools. *See,* e.g., Pei et al. 2006, Reynolds et al. 2004, Khvorova et al. 2003, Schwarz et al. 2003, Ui-Tei et al. 2004, Heale et al. 2005, Chalk et al. 2004, Amarzguioui et al. 2004.

In some embodiments, the polynucleotide is a miRNA. In some embodiments, the miRNA is about 22 nucleotides in length. miRNAs bind to target sites on mRNA molecules and silence the mRNA, e.g., by causing cleavage of the mRNA, destabilization of the mRNA, or inhibition of translation of the mRNA.

In some embodiments, the polynucleotide is a siRNA. In some embodiments, the siRNA comprises a nucleotide sequence that is identical to about a 15-25 contiguous mRNA sequence encoding the target protein. In some embodiments, the siRNA is a double-stranded RNA molecule having about 19-25 base pairs. In some embodiments, the siRNA commences with the dinucleotide AA. In some embodiments, the siRNA has a GC-content of about 30-70%, e.g., about: 30-65%, 30-60%, 30-55%, 30-50%, 40-70%, 40-65%, 40-60%, 40-55%, 45-70%, 45-65%, 45-60% or 45%-55%.

In some embodiments, the polynucleotide is a shRNA. A shRNA is an RNA molecule including a hairpin turn that decreases expression of target genes via RNAi. shRNAs can be delivered to cells in the form of plasmids, e.g., viral or bacterial vectors, e.g., by transfection, electroporation, or transduction.

siRNAs and shRNAs resemble intermediates in the processing pathway of the endogenous microRNA (miRNA) genes (*see,* e.g., Bartel, Cell 116:281-97 (2004)). In some embodiments, the siRNA functions as an miRNA; in other embodiments, the miRNA functions as an siRNA (*see,* e.g., Zeng et al., Mol Cell 9:1327-33 (2002); Doench et al., and Genes Dev 17:438-42 (2003)). MicroRNAs, like siRNAs, use RISC to downregulate target genes, but unlike siRNAs, most animal miRNAs do not cleave the mRNA. Instead, miRNAs reduce protein output through translational suppression or polyA removal and mRNA degradation (see, e.g., Wu et al., Proc Natl Acad Sci USA 103:4034-39 (2006)). Known miRNA binding sites are within mRNA 3' UTRs; miRNAs seem to target sites with near-perfect complementarity to nucleotides 2-8 from the miRNA's 5' end (*see,* e.g., Rajewsky, Nat Genet 38 Suppl: S8-13 (2006) and Lim et al., Nature 433:769-73 (2005)). This region is known as the seed region. Because siRNAs and miRNAs are interchangeable, exogenous siRNAs downregulate mRNAs with seed complementarity to the siRNA (*see,* e.g., Birmingham et al., Nat Methods 3:199-204 (2006)). Multiple target sites within a 3' UTR give stronger downregulation (*see,* e.g., Doench et al., Genes Dev 17: 438-42 (2003)).

In some embodiments, the polynucleotide is a messenger RNA (mRNA) or a circular RNA (circRNA) encoding a target protein disclosed herein or a variant thereof (e.g., a variant that is at least about 70% identical (e.g., at least about: 75%, 80%, 85%, 90%, 95%, 98% or 99% identical to the wild-type protein). In some embodiments, the mRNA is codon optimized (e.g., to improve efficacy of protein synthesis and limit mRNA destabilization by rare codons (*see,* e.g., Presnyak et al., Cell. 160 (6): 1111-24 (2015) and Thess et al., Mol Ther. 23(9): 1456-64 (2015)).

In some embodiments, a polynucleotide comprising RNA is chemically synthesized. In some embodiments, a polynucleotide comprising RNA is expressed recombinantly. In some embodiments, the RNA is transcribed *in vitro.* The making and using of RNA therapeutics are known in the art. *See,* for example, RNA Therapeutics: Function, Design, and Delivery (Mouldy Sioud eds., 2010) and Kaczmarek et al., Advances in the delivery of RNA therapeutics: from concept to clinical reality, Genome Medicine 9:60 (2017).

In some embodiments, the mRNA is produced by *in vitro* transcription. In some embodiments, the mRNA is modified to optimize its activity. In some embodiments, the mRNA comprises a modified base, a 5' cap, a 5' cap analogue, an anti-reverse cap analog (ARCA), or a combination thereof.

In some embodiments, the mRNA comprises a poly(A) tail. In some embodiments, the poly(A) tail is about 100-200 nucleotides. In some embodiments, the poly(A) tail improves the expression and/or stability of the mRNA (*see,* e.g., Kaczmarek et al., Genome Medicine 9:60 (2017)).

In some embodiments, the mRNA comprises 5' cap. In some embodiments, the mRNA comprises a 5' cap analogue. In some embodiments, the 5' cap analogue is a 1,2-dithiodiphosphate-modified cap (*see,* e.g., Strenkowska et al., Nucleic Acids Res. 44:9578-90 (2016)).

In some embodiments, the mRNA comprises a modified 3' untranslated region (UTR), a 5' UTR, or both. In some embodiments, the modified UTR comprises sequences responsible for recruiting RNA-binding proteins (RBPs) and miRNAs (e.g., to enhance the level of protein product (*see,* e.g., Kaczmarek et al., Genome Medicine 9:60 (2017)). In some embodiments, the 3' UTR, 5' UTR, or both are modified to encode a regulatory element. In some embodiments, the regulatory element comprises a K-turn motif, a miRNA binding site, or a combination thereof to control RNA expression in a cell-specific manner (*see,* e.g., Wroblewska et al., Nat Biotechnol. 33:839-41 (2015)).

In some embodiments, the mRNA comprises an RNA base modification. In some embodiments, the mRNA comprises a pseudouridine. In some embodiments, the mRNA comprises a N1-methyl-pseudouridine (e.g., to mask immune-stimulatory activity and enhance translation initiation (*see,* e.g., Andries et al., J Control Release 217:337-44 (2015) and Svitkin et al., Nucleic Acids Res. 45:6023-36 (2017)).

In some embodiments, the RNA (e.g., mRNA) is a circular RNA.

Compositions and methods for producing mRNA are disclosed, *see,* e.g., in WO2016011306, WO2016014846, WO2016022914, WO2016077123, WO2016164762, WO2016201377, WO2017049275, US9937233, US8710200, US10022425, US9878056, US9572897, WO2010084371, US9353153, WO2015034925 and WO2019236673. Also *see,* e.g., Jemielity et al., RNA 9(9):1108-22 (2003); Mockey et al., Biochem Biophys Res Commun. 340: 1062-88 (2006); Strenkowska et al. Nucleic Acids Res. 44: 9578-90 (2016); Presnyak et al., Cell 160: 1111-24 (2015) and Kaczmarek et al., Genome Medicine 9:60 (2017)). In some embodiments, the mRNA is prepared in a lipid nanoparticle (LNP) formulation (e.g., for *in vivo* delivery, *see,* e.g., US Pat. 9,764,036, US Pat. 9,682,139, Kauffman et al., Nano Lett. 15: 7300-6 (2015) and Fenton et al., Adv Mater. 28: 2939-43 (2016)).

In some embodiments, the polynucleotide is an aptamer. In certain embodiments, the aptamer binds to a target protein disclosed herein. In particular embodiments, the aptamer binds to a binding partner of a target protein disclosed herein.

In some embodiments, the polynucleotide is linked (e.g., covalently) to a delivery polymer. In some embodiments, the link between the polynucleotide and the delivery polymer is reversible. In some embodiments, the polynucleotide is linked to the delivery polymer via a physiologically labile linker. In some embodiments, the physiologically labile linker is a disulfide bond.

In some embodiments, the polynucleotide is conjugated to the polymer in the presence of an excess of polymer. In some embodiments, the excess polymer is removed prior to administration (e.g., to a cell or a subject).

A person of ordinary skill in the art can readily make suitable polynucleotide agents for use in the compositions, kits and methods described herein using the gene locus information of the protein sequences contained in the Sequence Listing incorporated herein, and Table A, such as the chromosomal location, starting and ending nucleotide positions, and polymorphism identifications.

### C. Agents Comprising Gene Editing Systems

In some embodiments, the agent comprises a gene editing system. In some embodiments, the gene editing system produces a deletion of nucleotides, a substitution of nucleotides, an addition of nucleotides or a combination of the foregoing, in a gene encoding a target protein.

In some embodiments, the gene editing system is a CRISPR/Cas system, a transposon-based gene editing system, or a transcription activator-like effector nuclease (TALEN) system. In some embodiments, the gene editing system is a CRISPR/Cas system. In some embodiments, the gene editing system is a class II CRISPR/Cas system.

In some embodiments, the gene editing system (e.g., the CRISPR/Cas system) reduces (e.g., decreases, inhibits) or eliminates (e.g., via gene knockout) the expression of the target protein. In some embodiments, the gene editing system (e.g., the CRISPR/Cas system) reduces (e.g., decreases, inhibits) or eliminates (e.g., via gene knockout) the expression of a protein capable of modulating the expression or activity of the target protein. In some embodiments, the gene editing system (e.g., the CRISPR/Cas system) increases (e.g., via gene knock-in or gene replacement) the expression of the target protein. In some embodiments, the gene editing system (e.g., the CRISPR/Cas system) increases (e.g., via gene knock-in or gene replacement) the expression of a protein capable of modulating the expression or activity of the target protein.

In some embodiments, the CRISPR system specifically catalyzes cleavage in a gene encoding the target protein, thereby inactivating said gene. Repairing nucleic acid strand breaks through non-homologous end joining (NHEJ) often results in changes to the DNA sequence at the site of the cleavage, resulting in small insertions or deletions (Indels). In some embodiments, NHEJ is used to knock out the gene encoding the target protein. In some embodiments, homology directed repair (HDR) is used to concurrently inactivate a gene encoding the target protein and insert a heterologous sequence into the inactivated locus. Cells in which a knockout and/or knockin event has occurred can be identified and/or selected by well-known methods in the art.

In some embodiments, the gene editing system comprises a single Cas endonuclease or a polynucleotide encoding the single Cas endonuclease. In some embodiments, the single Cas endonuclease is Cas9, Cpfl, C2C1 or C2C3. In some embodiments, the single Cas endonuclease is Cas9 (e.g., of *Streptococcus Pyogenes*). In some embodiments, the single Cas endonuclease is Cpfl. In some embodiments, the Cpf1 is AsCpf1 (from *Acidaminococcus sp.*) or LbCpf1 (from *Lachnospiraceae sp.*)*.* The choice of nuclease and gRNA(s) will typically be determined according to whether a deletion, a substitution, or an addition of nucleotide(s) to a targeted sequence is desired.

In some embodiments, the type II Cas endonuclease is Cas 9 (e.g., of *Streptococcuspyogenes*)*.* In some embodiments, the modified Cas 9 is nickase Cas9, dead Cas9 (dCas9) or eSpCas9. In some embodiments, the nickase Cas9 is Cas9 D10A. In some embodiments, the dCas9 is D10A or H840A. In some embodiments, the gene editing system comprises a double nickase Cas9 (e.g., to achieve more accurate genome editing, see, e.g., Ran et al., Cell 154: 1380-89 (2013). Wild-type Cas9 generates double-strand breaks (DSBs) at specific DNA sequences targeted by a gRNA. Nickase Cas9 generates only a single-strand break. dCas9 is catalytically inactive. In some embodiments, dCas9 is fused to a nuclease (e.g., a FokI to generate DSBs at target sequences homologous to two gRNAs). Various CRISPR/Cas9 plasmids are publicly available from the Addgene repository (Addgene, Cambridge, MA: addgene.org/crispr/).

In some embodiments, the gene editing system comprises:
a) a wild-type or modified type II Cas endonuclease or a polynucleotide encoding the wild-type or modified type II Cas endonuclease;
b) a CRISPR RNA ("crRNA"); and
c) a trans-activating crRNA ("tracrRNA").

In some embodiments, the crRNA comprises at least 1 "guide RNA" (sgRNA), e.g., at least: 2, 3 or 4 gRNAs. In some embodiments, the gRNA comprises a sequence that is identical to a portion of the gene sequence of the target protein. In some embodiments, the gRNA comprises a sequence that is identical to a portion of the gene sequence of a protein capable of modulating the expression or activity of the target protein. In some embodiments, the gRNA is at least about 16 nucleotides, e.g., at least about: 17, 18, 19, 20, 21, 22, 23, 24 or 25 nucleotides; or about: 16, 17, 18, 19, 20, 21, 22, 23, 24 or 25 nucleotides; or about: 16-24, 17-24, 17-23, 18-23, 18-22, 19-22 or 19-21 or 19, 20 or 21 nucleotides. In some embodiments, the sgRNA is chemically modified.

Design of gRNA sequences for gene editing are known in the art. *See,* for example, Cong et al., Science, 339: 819-23 (2013) and Ran et al., Nature Protocols 8: 2281-308 (2013). Cas9 requires at least about 16 or 17 nucleotides of gRNA sequence to cleave DNA, and Cpf1 requires at least about 16 nucleotides of gRNA sequence to cleave DNA. In practice, a gRNA sequence has a length of about 17-24 nucleotides (e.g., about: 19, 20 or 21 nucleotides) and is complementary to a target gene. Custom gRNA generators and algorithms are commercially available. Chemically modified sgRNAs have also been demonstrated to be effective in genome editing (*see,* e.g., Hendel et al., Nature Biotechnol., 985-91 (2015)).

In some embodiments, the crRNA further comprises a sequence capable of binding to the tracrRNA. Upon binding, the partially double-stranded structure is cleaved by RNase III, the resulting crRNA/tracrRNA hybrid directs the Cas9 endonuclease to recognize and cleave a target DNA sequence.

In some embodiments, the target DNA sequence is approximate to a "protospacer adjacent motif" ("PAM") that is specific for a Cas endonuclease. PAM sequences appear throughout a given genome. CRISPR endonucleases of various prokaryotic species have unique PAM sequence requirements. Non-limiting examples of PAM sequences include: 5'-NGG (Streptococcus pyogenes), 5'-NNAGAA (Streptococcus thermophilus CRISPR1), 5'-NGGNG (Streptococcus thermophilus CRISPR3) and 5'-NNNGATT (Neisseria meningiditis). Some endonucleases, e.g., Cas9 endonucleases, are associated with G-rich PAM sites, e.g., 5'-NGG, and perform blunt-end cleaving of the target DNA at a location that is 3 nucleotides upstream (5') of the PAM site.

In some embodiments, the gene editing system comprises:
a) a wild-type or modified type II Cas endonuclease or a polynucleotide encoding the wild-type or modified type II Cas endonuclease; and
b) a crRNA.

Cpfl-associated CRISPR arrays are processed into mature crRNAs without the requirement of a tracrRNA. Cpf1 endonucleases, are associated with T-rich PAM sites, e.g., 5'-TTN. Cpfl can also recognize a 5'-CTA PAM motif. Cpfl cleaves the target DNA by introducing an offset or staggered double-strand break with a 4- or 5-nucleotide 5' overhang, for example, cleaving a target DNA with a 5-nucleotide offset or staggered cut located 18 nucleotides downstream (3') of the PAM site on the coding strand and 23 nucleotides downstream from the PAM site on the complimentary strand. The 5-nucleotide overhang that results from such offset cleavage allows more precise genome editing by DNA insertion by homologous recombination than by insertion at blunt-end cleaved DNA. *See,* e.g., Zetsche et al., Cell 163:759-71 (2015).

In some embodiments, the gene editing system activates or represses transcription of a target gene. In some embodiments, the gene editing system comprises:
a) a chimeric protein comprising dCas9 and one or more effector domains; and
b) one or more sgRNAs.

In some embodiments, the chimeric protein represses the expression of the target protein (CRISPRi). In some embodiments, the chimeric protein activates the expression of the target protein (CRISPRa). In some embodiments, the chimeric protein methylates a DNA sequence recognized by the sgRNA. In some embodiments, the chimeric protein demethylates a DNA sequence recognized by the sgRNA.

An effector domain comprises a biologically active portion of an effector protein (e.g., transcriptional activator or repressor). In some embodiments, the gene editing system comprises 1 effector domain. In some embodiments, the gene editing system comprises at least 2 effector domains, e.g., 2, 3 or 4 effector domains. In some embodiments, the effector domain comprises KRAB. In some embodiments, the effector domain comprises VP64. In some embodiments, the effector domain comprises VP64, p65 and Rta. In some embodiments, the dCas9 is D10A. In some embodiments, the dCas9 is H840A.

Because dCas9 is catalytically inactive, dCas9 does not cut the target DNA but interferes with transcription by steric hindrance. The dCas9 chimeric protein (e.g., dCas9-VPR), guided by the one or more gRNAs to the upstream sequence of a transcriptional start site (TSS) of the target gene, modulates transcription of the target gene. *See,* e.g., Gilbert et al., CRISPR-Mediated Modular RNA-Guided Regulation of Transcription in Eukaryotes, Cell 154, 442-51 (2013); Cheng et al., Multiplexed activation of endogenous genes by CRISPR-on, an RNA-guided transcriptional activator system, Cell Res. 23: 1163-71 (2013); Gilbert et al., Genome-Scale CRISPR-Mediated Control of Gene Repression and Activation, Cell 159: 647-61 (2014); Tanenbaum et al., A protein-tagging system for signal amplification in gene expression and fluorescence imaging, Cell 159: 635-46 (2014); Konermann et al., Genome-scale transcriptional activation by an engineered CRISPR-Cas9 complex, Nature 517: 583-88 (2015); Chavez et al., Highly efficient Cas9-mediated transcriptional programming, Nat. Methods. 12: 326-28 (2015); Zalatan et al., Engineering complex synthetic transcriptional programs with CRISPR RNA scaffolds, Cell 160: 339-50 (2015); Horlbeck et al., Compact and highly active next-generation libraries for CRISPR-mediated gene repression and activation, eLife. 5: e19760 (2016); Chavez et al., Comparison of Cas9 activators in multiple species, Nat Methods. 7: 563-67 (2016).

CRISPR technology for editing the genes of eukaryotes is disclosed in US Patent Application Publications 2016/0138008A1 and US2015/0344912A1, and in US Patents 8,697,359, 8,771,945, 8,945,839, 8,999,641, 8,993,233, 8,895,308, 8,865,406, 8,889,418, 8,871,445, 8,889,356, 8,932,814, 8,795,965, and 8,906,616. Cpfl endonuclease and corresponding guide RNAs and PAM sites are disclosed in US Patent Application Publication 2016/0208243 A1. CRISPR technology for generating mtDNA dysfunction in the mitochondrial genome is disclosed in Jo et al., BioMed Res. Int. 2015: 305716 (2015). Co-delivery of Cas9 and sgRNA with nanoparticles is disclosed in Mout et al., ACS Nano 11(3): 2452-58 (2017).

In some embodiments, the agent comprises a transposon-based gene editing system. An example of a suitable transposon-based gene editing system for use in the disclosure provided herein is the Gene Writer system described in International Publication Number WO 2020/047124, published on March 5, 2020, the contents of which are incorporated herein by referenced in their entirety.

In some embodiments, the agent comprises a transcription activator-like effector nuclease (TALEN) system. TALEN-based systems comprise a protein comprising a TAL effector DNA binding domain and an enzymatic domain. They are made by fusing a TAL effector DNA-binding domain to a DNA cleavage domain (a nuclease which cuts DNA strands). The FokI restriction enzyme described above is an exemplary enzymatic domain suitable for use in TALEN-based gene-regulating systems.

TAL effectors are proteins that are secreted by Xanthomonas bacteria via their type III secretion system when they infect plants. The DNA binding domain contains a repeated, highly conserved, 33-34 amino acid sequence with divergent 12th and 13th amino acids. These two positions, referred to as the Repeat Variable Diresidue (RVD), are highly variable and strongly correlated with specific nucleotide recognition. Therefore, the TAL effector domains can be engineered to bind specific target DNA sequences by selecting a combination of repeat segments containing the appropriate RVDs. The nucleic acid specificity for RVD combinations is as follows: HD targets cytosine, NI targets adenine, NG targets thymine, and NN targets guanine (though, in some embodiments, NN can also bind adenine with lower specificity)

In some embodiments, the TAL effector domains bind to a target DNA sequence that is at least 90%, 95%, 96%, 97%, 98%, or 99% identical, or is 100% identical to a target DNA sequence of the target protein. In some embodiments, the TAL effector domains bind to a target DNA sequence that is at least 90%, 95%, 96%, 97%, 98%, or 99% identical, or is 100% identical to a target DNA sequence of a target protein defined by a set of genomic coordinates.

In some embodiments, the gene-regulating system comprises two or more TAL effector-fusion proteins each comprising a TAL effector domain, wherein at least one of the TAL effector domains binds to a target DNA sequence of the target protein. In some embodiments, at least one of the two or more TAL effector domains binds to a target DNA sequence that is at least 90%, 95%, 96%, 97%, 98%, or 99% identical, or is 100% identical to a target DNA sequence defined by a set of genomic coordinates.

Methods and compositions for assembling the TAL-effector repeats are known in the art. See e.g., Cermak et al, Efficient design and assembly of custom TALEN and other TAL effector-based constructs for DNA targeting, Nucleic Acids Res 39(12): e82 (2011). Plasmids for constructions of the TAL-effector repeats are commercially available from e.g., Addgene.

In some embodiments, the agent comprises a zinc finger nuclease (ZFN) system. ZFN domains can be generated using commercially available plasmids, such as, for example, plasmid pairs (CSTZFN-1KT COMPOZR^{®} Custom Zinc Finger Nuclease (ZFN) R-3257609) from Sigma Aldrich (St. Louis, MO). Plasmids can be prepared using the commercial system following manufacturer's protocol (e.g., NEB Monarch Miniprep (Cat# T1010), New England Biolabs, Ipswich, MA).

In some embodiments, the agent comprises a vector designed for delivering conventional gene therapy (e.g., gene knockout or knock-in via homologous recombination). Non-limiting examples of said vectors include retrovirus (e.g., Lentivirus 5), adenovirus, adeno-associated virus, Herpes Simplex Virus, nanoparticles and DNA Transposons.

### D. Small Molecule Agents

In some embodiments, the agent comprises a small molecule. In some embodiments, the small molecule binds to the target protein. In some embodiments, the small molecule binds to a protein capable of modulating the expression or activity of the target protein. In some embodiments, the small molecule is an inhibitor of the target protein (e.g., a direct inhibitor, an indirect inhibitor). In some embodiments, the small molecule is an activator of the target protein (e.g., a direct activator, and indirect activator).

Examples of small molecules include organic compounds, organometallic compounds, inorganic compounds, and salts of organic, organometallic or inorganic compounds. Atoms in a small molecule are typically linked together via covalent and/or ionic bonds. In certain embodiments, the small molecule is a small organic molecule. The arrangement of atoms in a small organic molecule may represent a chain (*e.g.* a carbon-carbon chain or a carbon-heteroatom chain), or may represent a ring containing carbon atoms, *e.g.* benzene or a polycyclic system, or a combination of carbon and heteroatoms, *i.e.,* heterocycles such as a pyrimidine or quinazoline. Although small molecules can have a wide range of molecular weights, they generally include molecules that are less than about 5,000 daltons. For example, such small molecules can be less than about 1000 daltons and, preferably, are less than about 750 daltons or, more preferably, are less than about 500 daltons. Small molecules can be found in nature (*e.g*., identified, isolated, purified) and/or produced synthetically (*e.g*., by traditional organic synthesis, bio-mediated synthesis, or a combination thereof). See *e.g*. Ganesan, Drug Discov. Today 7(1): 47-55 (January 2002); Lou, Drug Discov. Today, 6(24): 1288-1294 (December 2001). Examples of naturally occurring small molecules include, but are not limited to, hormones, neurotransmitters, nucleotides, amino acids, sugars, lipids, and their derivatives.

In particular embodiments, the agent comprises a proteolysis targeting chimera (PROTAC).

A small molecule suitable for use in the compositions, kits and methods of this disclosure can be identified by a person of ordinary skill in the art using any of the screening methods disclosed herein.

### E. Therapeutic Cells and Cell-Based Therapies

In some embodiments, the agent comprises a therapeutic cell. In certain embodiments, the therapeutic cell expresses and/or is engineered to express a target protein (e.g., a target protein in the Sequence Listing, Table A, or a variant thereof), a polypeptide (e.g., an antibody, an antigen-binding fragment or a polypeptide that comprises an amino acid sequence that is at least 70% identical to at least a portion of the target protein), a polynucleotide (e.g., a recombinant DNA, an RNA such as mRNA or siRNA), and/or a gene editing system (e.g., a CRISPR/Cas system) described herein.

In some embodiments, a polypeptide disclosed herein (e.g., an antibody or antigen-binding fragment) is incorporated into a cell-based therapy. In some embodiments, the polypeptide is an engineered T cell receptor. In some embodiments, the polypeptide is a chimeric antigen receptor (CAR) (e.g., expressed on a T (CAR-T) cell, natural killer (CAR-NK) cell, or macrophage (CAR-M) cell). In some embodiments, the CAR comprises a transmembrane domain and an antigen-recognition moiety, wherein the antigen-recognition moiety binds the target protein.

A therapeutic cell suitable for use in the compositions, kits and methods of this disclosure can be generated, identified and/or enriched with methods known to a person of ordinary skill in the art. Non-limiting examples of said methods include purifying, propagating and/or differentiating cells from a subject (e.g., a human) to a specific cell product; engineering a somatic cell for gene therapy; cell immortalization; *ex vivo* gene modification of a cell (e.g., using viral vector and/or lipid nanoparticle delivery technologies); *in vivo* gene modification of a cell (e.g., using viral vector and/or lipid nanoparticle delivery technologies); genome editing; cell plasticity technologies; gene modifications; and flow cytometry. In some embodiments, the therapeutic cell is autologous or syngeneic. In other embodiments, the therapeutic cell is allogeneic.

### Expression Vectors and Hosts

In another aspect, the disclosure provides an expression vector comprising a polynucleotide described herein.

The term "expression vector" refers to a replicable nucleic acid from which one or more proteins can be expressed when the expression vector is transformed into a suitable expression host cell.

In some embodiments, the expression vector comprises an expression control polynucleotide sequence operably linked to the polynucleotide, a polynucleotide sequence encoding a selectable marker, or both. In some embodiments, the expression control polynucleotide sequence comprises a promoter sequence, an enhancer sequence, or both. In some embodiments, the expression control polynucleotide sequence comprises an inducible promoter sequence. The term "promoter" refers to a region of DNA to which RNA polymerase binds and initiates the transcription of a gene. The term "operably linked" means that the nucleic acid is positioned in the recombinant polynucleotide, e.g., vector, in such a way that enables expression of the nucleic acid under control of the element (e.g., promoter) to which it is linked. The term "selectable marker element" is an element that confers a trait suitable for artificial selection. Selectable marker elements can be negative or positive selection markers. Non-limiting examples of expression vectors for use with bacterial, fungal, yeast, and mammalian cellular hosts are described in Molecular Cloning: A Laboratory Manual (Michael R. Green & Joseph Sambrook eds., 4th ed. 2012).

In another aspect, the disclosure provides an expression host cell comprising any one or more of the polynucleotides or expression vectors described herein.

The term "expression host cell" refers to a cell useful for receiving, maintaining, reproducing and/or amplifying a vector.

Non-limiting examples of expression host cells include mammalian cells such as hybridoma cells, Baby Hamster Kidney fibroblasts (BHK cells), Chinese hamster ovary (CHO) cells, COS cells, HeLa cells, and human embryonic kidney (HEK), yeast cells such as *Pichia pastoris* cells, or bacterial cells such as DH5α, etc. *See,* e.g., Mammalian Cell Cultures for Biologics Manufacturing (Weichang Zhou & Anne Kantardjieff eds., 2014) for processes of host cell culture for production of protein therapeutics; Protein Biotechnology: Isolation, Characterization, and Stabilization (Felix Franks eds., 2013) and Protein Purification Protocols (Paul Cutler eds., 2010) for purification of protein therapeutics; and Therapeutic Protein Drug Products: Practical Approaches to formulation in the Laboratory, Manufacturing, and the Clinic (Brian K Meyer eds., 2012) for formulation of protein therapeutics.

A polynucleotide or expression vector described herein can be introduced into a suitable or desired host cell using techniques known in the art, including transformation, electroporation, and transduction. The introduced nucleic acid can be extrachromosomal in the host cell, or integrated into the host cell's genome.

### Pharmaceutical Compositions

In another aspect, the disclosure provides a pharmaceutical composition, wherein the pharmaceutical composition comprises an agent disclosed herein, and a pharmaceutically acceptable carrier. As used herein, the term "pharmaceutical composition" refers to a composition having pharmacological activity or other direct effect in mitigating, treating, or preventing cancer, or a finished dosage form or formulation thereof.

In some embodiments, the composition (e.g., pharmaceutical composition) comprises pharmaceutically acceptable carriers, excipients, stabilizers, diluents or tonifiers (Remington's Pharmaceutical Sciences 16th edition, Osol, A. Ed. (1980)). Suitable pharmaceutically acceptable carriers, excipients, or stabilizers are nontoxic to recipients at the dosages and concentrations employed. Non-limiting examples of pharmaceutically acceptable carriers, excipients, stabilizers, diluents or tonifiers include buffers (e.g., phosphate, citrate, histidine), antioxidants (e.g., ascorbic acid or methionine), preservatives, proteins (e.g., serum albumin, gelatin or immunoglobulins); hydrophilic polymers, amino acids, carbohydrates (e.g., monosaccharides, disaccharides, glucose, mannose or dextrins); chelating agents (e.g., EDTA), sugars (e.g., sucrose, mannitol, trehalose or sorbitol), salt-forming counter-ions (e.g., sodium), metal complexes (e.g., Zn-protein complexes); non-ionic surfactants (e.g., Tween), PLURONICS^{™} and polyethylene glycol (PEG).

In some embodiments, the agent of the pharmaceutical compositions (e.g., polypeptide, polynucleotide, or small molecule) is modified, e.g., conjugated to a heterologous moiety. The term "conjugated" refers to attached, via a covalent or noncovalent interaction. Conjugation can employ any of suitable linking agents; non-limiting examples include peptide linkers, compound linkers, and chemical cross-linking agents.

In some embodiments, the heterologous moiety is a marker (e.g., a fluorescent or radioactive marker), a molecule that stabilizes the agent, a molecule that targets the agent (e.g., to a particular cell or tissue, to facilitate or prevent from crossing the blood brain barrier), or a combination thereof.

In some embodiments, the heterologous moiety is polyethylene glycol (PEG), hexadecanoic acid, hydrogels, nanoparticles, multimerization domains and carrier peptides. In some embodiments, the nanoparticle is a lipid nanoparticle. In some embodiments, the nanoparticle is a polymer nanoparticle. In some embodiments, the polymer is an amphiphilic polymer. In other embodiments, the polymer is a hydrophobic or hydrophilic polymer. Non-limiting examples of polymers include poly(lactic acid)-poly(ethylene glycol), poly(lactic-co-glycolic acid)-poly(ethylene glycol), poly(lactic-co-glycolic) acid (PLGA), poly(lactic-co-glycolic acid)-d-α-tocopheryl polyethylene glycol succinate, poly(lactic-co-glycolic acid)-ethylene oxide fumarate, poly(glycolic acid)-poly(ethylene glycol), polycaprolactone-poly(ethylene glycol), or any salts thereof. In some embodiments, the polymer nanoparticle comprises poly(lactic-co-glycolic) acid (PLGA).

In some embodiments, the composition (e.g., pharmaceutical composition) is formulated for a suitable administration schedule and route. Non-limiting examples of administration routes include oral, rectal, mucosal, intravenous, intramuscular, subcutaneous and topical, etc. In some embodiments, the composition (e.g., pharmaceutical composition) is stored in the form of an aqueous solution or a dried formulation (e.g., lyophilized). In some embodiments, the composition is formulated to be administered by infusion (e.g., intravenous infusion).

In some embodiments, the composition is formulated to be administered with one or more additional therapeutic agents (e.g., with a second therapeutic agent) as a combination therapy. As used herein, a "combination therapy" or "administered in combination" means that two (or more) different agents or treatments are administered to a subject as part of a defined treatment regimen for a particular disease or condition. Non-limiting examples of additional agents or treatments include biologics (e.g., antibodies, peptides), cell therapies, gene therapies, immunotherapies, and small molecules used in oncology (e.g., chemotherapeutic agents).

The treatment regimen defines the doses and periodicity of administration of each agent such that the effects of the separate agents on the subject overlap. In some embodiments, the two or more agents are administered in a sequential manner as part of a prescribed regimen. In other embodiments, the delivery of the two or more agents is simultaneous or concurrent. In some embodiments, the two or more agents are co-formulated. In some embodiments, administration of two or more agents or treatments in combination is such that the reduction in a symptom, or other parameter related to the disorder is greater than what would be observed with one agent or treatment delivered alone or in the absence of the other. The effect of the two treatments can be partially additive, wholly additive, or greater than additive (e.g., synergistic). Each of the two or more therapeutic agents can be administered by any appropriate route including, but not limited to, oral routes, intravenous routes, intramuscular routes, and direct absorption through mucous membrane tissues. The two or more therapeutic agents can be administered by the same route or by different routes.

In some embodiments, the agent or pharmaceutical composition of the disclosure is delivered by a viral vector, e.g., by contacting a cell with a viral vector, locally administered (e.g., injected) to a tumor, or systemically (e.g., intravenously or orally) administered to a subject (e.g., a human patient).

Viral genomes provide a rich source of vectors that can be used for the efficient delivery of exogenous genes into a mammalian cell. Viral genomes are particularly useful vectors for gene delivery because the polynucleotides contained within such genomes are typically incorporated into the nuclear genome of a mammalian cell by generalized or specialized transduction. These processes occur as part of the natural viral replication cycle, and do not require added proteins or reagents to induce gene integration. Non-limiting examples of viral vectors include retrovirus (e.g., Retroviridae family viral vector), adenovirus (e.g., Ad5, Ad26, Ad34, Ad35, and Ad48), parvovirus (e.g., adeno-associated viruses), coronavirus, negative strand RNA viruses such as orthomyxovirus (e.g., influenza virus), rhabdovirus (e.g., rabies and vesicular stomatitis virus), paramyxovirus (e.g., measles and Sendai), positive strand RNA viruses, such as picornavirus and alphavirus, and double stranded DNA viruses including adenovirus, herpesvirus (e.g., Herpes Simplex virus types 1 and 2, Epstein-Barr virus, cytomegalovirus, replication deficient herpes virus), and poxvirus (e.g., vaccinia, modified vaccinia Ankara (MVA), fowlpox and canarypox). Additional non-limiting examples include Norwalk virus, togavirus, flavivirus, reoviruses, papovavirus, hepadnavirus, human papilloma virus, human foamy virus, and hepatitis virus, for example. Non-limiting examples of retroviruses include: avian leukosis-sarcoma, avian C-type viruses, mammalian C-type, B-type viruses, D-type viruses, oncoretroviruses, HTLV-BLV group, lentivirus, alpharetrovirus, gammaretrovirus, spumavirus (*see,* e.g., Coffin JM. Retroviridae: The viruses and their replication. In: Fields BN, Knipe DM, Howley PM et al, eds. Fundamental Virology. 3rd ed. Philadelphia: Lippincott-Raven Publishers, 1996:763-843). Additional non-limiting examples include murine leukemia viruses, murine sarcoma viruses, mouse mammary tumor virus, bovine leukemia virus, feline leukemia virus, feline sarcoma virus, avian leukemia virus, human T-cell leukemia virus, baboon endogenous virus, Gibbon ape leukemia virus, Mason Pfizer monkey virus, simian immunodeficiency virus, simian sarcoma virus, Rous sarcoma virus and lentiviruses. Additional non-limiting examples of vectors are described, for example, in US Patent No. 5,801,030, the teachings of which are incorporated herein by reference.

In some embodiments, the agent or pharmaceutical composition of the disclosure is formulated to be delivered by a membrane-based carrier *in vivo, in vitro, ex vivo,* or *in situ.* In some embodiments, the membrane-based carrier is a cell-based carrier (e.g., mammalian such as human cells). In some embodiments, the membrane-based carrier is a vesicle-based carrier. In some embodiments, the membrane-based carrier comprises one or more vectors described herein (e.g., a plasmid, virus, viral-like particle or a virosome).

In some embodiments, the agent or pharmaceutical composition of the disclosure is formulated to be delivered by one or more liposomes. Liposomes are spherical vesicle structures composed of a uni- or multilamellar lipid bilayer surrounding internal aqueous compartments and a relatively impermeable outer lipophilic phospholipid bilayer. Liposomes may be anionic, neutral or cationic. Liposomes are biocompatible, nontoxic, can deliver both hydrophilic and lipophilic drug molecules, protect their cargo from degradation by plasma enzymes, and transport their load across biological membranes and the blood brain barrier (BBB) (*see,* e.g., Spuch and Navarro, J Drug Deliv. 2011: 469679 (2011)).

Vesicles can be made from several different types of lipids; however, phospholipids are most commonly used to generate liposomes as drug carriers. Methods for preparation of multilamellar vesicle lipids are known in the art (see for example U.S. Pat. No. 6,693,086, the teachings of which relating to multilamellar vesicle lipid preparation are incorporated herein by reference). Although vesicle formation can be spontaneous when a lipid film is mixed with an aqueous solution, it can also be expedited by applying force in the form of shaking by using a homogenizer, sonicator, or an extrusion apparatus (*see,* e.g., Spuch and Navarro, J Drug Deliv. 2011: 469679 (2011)). Extruded lipids can be prepared by extruding through filters of decreasing size, as described in Templeton et al., Nature Biotech, 15: 647-52 (1997), the teachings of which relating to extruded lipid preparation are incorporated herein by reference.

In some embodiments, the agent or pharmaceutical composition of the disclosure is formulated to be delivered by lipid nanoparticles (LNPs). In one embodiment, the LNP preparation comprising the agent or pharmaceutical composition of the disclosure has one or more of the following characteristics: (a) the LNP preparation comprises a cationic lipid, a neutral lipid, a cholesterol, and a PEG lipid, (b) the LNP preparation has a mean particle size of between 80 nm and 160 nm.

Nanostructured lipid carriers (NLCs) are modified solid lipid nanoparticles (SLNs) that retain the characteristics of the SLN, improve drug stability and loading capacity, and prevent drug leakage. Polymer nanoparticles (PNPs) are an important component of drug delivery. These nanoparticles can effectively direct drug delivery to specific targets and improve drug stability and controlled drug release. Lipid-polymer nanoparticles (PLNs), a new type of carrier that combines liposomes and polymers, may also be employed. These nanoparticles possess the complementary advantages of PNPs and liposomes. A PLN is composed of a core-shell structure; the polymer core provides a stable structure, and the phospholipid shell offers good biocompatibility. As such, the two components increase the drug encapsulation efficiency rate, facilitate surface modification, and prevent leakage of water-soluble drugs. *See,* e.g., Li et al., Nanomaterials 7(6): 122 (2017).

In some embodiments, the agent or pharmaceutical composition of the disclosure is formulated to be delivered by a carbohydrate carrier (e.g., an anhydride- modified phytoglycogen or glycogen-type material). Non-limiting examples of carbohydrate carriers include phytoglycogen octenyl succinate, phytoglycogen beta-dextrin and anhydride-modified phytoglycogen beta-dextrin.

In some embodiments, the agent or pharmaceutical composition of the disclosure is formulated to be delivered by a protein carrier (e.g., a protein covalently linked to the circular polyribonucleotide). Non-limiting examples of protein carriers include human serum albumin (HSA), low-density lipoprotein (LDL), high- density lipoprotein (HDL) and globulin.

In some embodiments, the agent or pharmaceutical composition of the disclosure is formulated to be delivered by a cationic carrier (e.g., a cationic lipopolymer or transfection reagent). Non-limiting examples of cationic carriers include lipofectamine, polyethylenimine, poly(trimethylenimine), poly(tetramethylenimine), polypropylenimine, aminoglycoside-polyamine, dideoxy-diamino-b-cyclodextrin, spermine, spermidine, poly(2-dimethylamino)ethyl methacrylate, poly(lysine), poly(histidine), poly(arginine), cationized gelatin, dendrimers, chitosan, 1,2-Dioleoyl-3- Trimethylammonium-Propane(DOTAP), N-[1-(2,3-dioleoyloxy) propyl]-N,N,N- trimethylammonium chloride (DOTMA), 1-[2-(oleoyloxy)ethyl]-2-oleyl-3-(2- hydroxyethyl)imidazolinium chloride (DOTIM), 2,3-dioleyloxy-N-[2(sperminecarboxamido) ethyl]-N,N-dimethyl-1-propanaminium trifluoroacetate (DOSPA), 3B-[N- (N\N'-Dimethylaminoethane)-carbamoyl]Cholesterol Hydrochloride (DC-Cholesterol HC1), diheptadecylamidoglycyl spermidine (DOGS), N,N-distearyl-N,N- dimethylammonium bromide (DDAB), N-(1,2-dimyristyloxyprop-3-yl)-N,N-dimethyl-N- hydroxyethyl ammonium bromide (DMRIE) and N,N-dioleyl-N,N-dimethylammonium chloride (DODAC).

In some embodiments, the agent or pharmaceutical composition of the disclosure is formulated to be delivered by exosomes, adipocytes and/or red blood cells. *See,* e.g., Ha et al., Acta Pharm Sin B. 6(4): 287-96 (2016).

In some embodiments, the agent or pharmaceutical composition of the disclosure is formulated to be delivered by one or more Fusosomes. Fusosomes have been engineered to confer target cell specificity for the fusion and payload delivery, allowing the creation of delivery vehicles with programmable cell specificity. See, e.g., Patent Application WO2020014209, the teachings of which relating to fusosome design, preparation, and usage are incorporated herein by reference.

In some embodiments, the agent or pharmaceutical composition of the disclosure is formulated to be delivered by *ex vivo* differentiated red blood cells. See, e.g., WO2015073587; WO2017123646; WO2017123644; WO2018102740; WO2016183482; WO2015153102; WO2018151829; WO2018009838; Shi et al., PNAS, 111(28): 10131-36 (2014); US Patent 9,644,180; Huang et al., Nature Communications 8: 423 (2017).

In some embodiments, the agent or pharmaceutical composition of the disclosure is formulated to be delivered by one or more microsomes, virus-like particles (VLPs) or plant nanovesicles and plant messenger packs (PMPs). *See,* e.g., WO2011097480, WO2013070324, WO2017004526 and WO2020041784.

In some embodiments, the agent or pharmaceutical composition of the disclosure is formulated to be delivered by one or more anellosomes. The making and use of anellosomes used for delivery of therapeutic products is described in US Pat. 11,166,996, the teachings of which relating to anellosome design, preparation, and usage are incorporated herein by reference.

### Methods

In another aspect, the disclosure provides a method of detecting a cancer or predicting a likelihood of (or risk level for) developing cancer in a subject, comprising quantifying an expression or activity of a target protein in a sample from the subject, wherein the level of expression or activity of the target protein in the sample is indicative of the likelihood of developing cancer in the subject.

In another aspect, the disclosure provides a method of classifying a subject based on a predicted likelihood of developing cancer, comprising quantifying an expression or activity of a target protein in a sample from the subject; predicting the likelihood of developing cancer based on the expression or activity of the target protein in the sample; and classifying the patient based on the predicted likelihood.

In another aspect, the disclosure provides a method of stratifying a set of subjects having cancer, comprising: quantifying an expression and/or activity of a target protein in samples from individual subjects in the set; and stratifying the set of subjects for treatment according to the individual subjects' levels of the expression and/or activity of the target protein in the samples.

In some embodiments, a higher expression or activity level of a target protein in a sample from the subject, relative to an appropriate control (e.g., reference standard) is indicative of the cancer or a likelihood of developing the cancer. In some embodiments, a lower expression or activity level of a target protein in a sample from the subject, relative to an appropriate control (e.g., reference standard) is indicative of the cancer or a likelihood of developing the cancer.

In some embodiments, the method further comprises administering to a subject who is determined to have or predicted to have a likelihood (or be at risk) of developing cancer, an effective amount of an agent disclosed herein or a pharmaceutical composition disclosed herein.

In some embodiments, the method further comprises administering to a subject who is determined to have or predicted to have a likelihood of developing cancer, an effective amount of an agent disclosed herein or a pharmaceutical composition disclosed herein.

In another aspect, the disclosure provides a method of preparing a sample that is useful for detecting a likelihood of developing cancer in a subject, comprising:
a) obtaining or having obtained a sample from the subject;
b) adding a protease inhibitor, a control peptide, a standard peptide, or a combination thereof to the sample to prepare a sample that is useful for detecting a likelihood of developing cancer; and
c) quantifying an expression or activity of a target protein in the sample prepared in step b).

In another aspect, the disclosure provides a method of treating a cancer in a subject in need thereof, comprising administering to the subject an effective amount of an agent disclosed herein or a pharmaceutical composition disclosed herein.

In another aspect, the disclosure provides a method of treating a cancer in a subject in need thereof, comprising administering to the subject an effective amount of an agent disclosed herein or a pharmaceutical composition disclosed herein, wherein the subject has an altered level of expression and/or activity of a target protein disclosed herein.

"Treatment" and "treating," as used herein, refer to the medical management of a subject with the intent to improve, ameliorate, stabilize (i.e., not worsen), prevent or cure a disease, pathological condition, or disorder. "Treatment" includes active treatment (treatment directed to improve the disease, pathological condition, or disorder), causal treatment (treatment directed to the cause of the associated disease, pathological condition, or disorder), palliative treatment (treatment designed for the relief of symptoms), preventative treatment (treatment directed to minimizing or partially or completely inhibiting the development of the associated disease, pathological condition, or disorder); and supportive treatment (treatment employed to supplement another therapy). Treatment also includes diminishing the extent of the disease or condition; preventing spread of the disease or condition; delaying or slowing the progress of the disease or condition; ameliorating or palliating the disease or condition; and remission (whether partial or total), whether detectable or undetectable. "Ameliorating" or "palliating" a disease or condition means that the extent and/or undesirable clinical manifestations of the disease, disorder, or condition are lessened and/or time course of the progression is slowed or lengthened, as compared to the extent or time course in the absence of treatment. "Treatment" also includes prolonging survival as compared to expected survival if not receiving treatment. Those in need of treatment include those already with the condition or disorder, as well as those prone to have the condition or disorder or those in which the condition or disorder is to be prevented.

In some embodiments, the subject is an animal. In other embodiments the subject is a bird, e.g., a hen, rooster, turkey or parrot. In some embodiments, the subject is a mammal. In some embodiments the subject is a non-human mammal. Non-limiting examples of a non-human mammal include cattle (e.g., dairy or beef cattle), sheep, goat, pig, horse, dog, cat, mouse, rat, etc. In some embodiments, the subject is a human. In some embodiments, the human is a neonate. In some embodiments, the human is a pediatric patient. In some embodiments, the human is a juvenile. In some embodiments, the human is an adult. In some embodiments, the human is less than 18 years old. In some embodiments, the human is at least 18 years old. In some embodiments, the human is between 18 and 25 years old. In some embodiments, the human is at least 25 years old, e.g., at least 30, 35, 40, 45, 50, 55, 60, 65, 70, 75, or 80 years old.

As used herein, the term "effective amount," "therapeutically effective amount," or "sufficient amount" refers to a quantity sufficient to, when administered to a subject (e.g., a mammal such as a human cancer patient), effect treatment (e.g., produce beneficial or desired results), including effects at cellular, tissue or clinical levels, etc. As such, the term depends upon the context in which it is being applied. For example, in the context of treating cancer, it is an amount of an agent sufficient to achieve a response as compared to the response obtained without administration of the agent. The amount of a given composition described herein that will correspond to such an amount will vary depending upon various factors, such as the given agent, the pharmaceutical formulation, the route of administration, the type of disease or disorder, the identity of the subject (e.g., age, sex, weight) or host being treated, and the like, but can nevertheless be routinely determined by one skilled in the art. In some embodiments, a "therapeutically effective amount" of a composition of the present disclosure is an amount that results in a beneficial or desired result in a subject (e.g., as compared to a control). A therapeutically effective amount of a composition of the present disclosure may be readily determined by one of ordinary skill by routine methods known in the art. Dosage regimen may be adjusted to provide the optimum therapeutic response.

In some embodiments, the effective amount is sufficient to reduce cancer (e.g., tumor) growth, proliferation, metastasis, invasion, migration, innervation, or a combination of the foregoing. In certain embodiments, said reduction is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, said reduction is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

In certain embodiments, the effective amount is sufficient to reduce expression of the target protein. In some embodiments, said reduction is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, said reduction is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

In certain embodiments, the effective amount is sufficient to increase expression of the target protein. In some embodiments, said increase is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, said increase is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%. In some embodiments, said increase is by about 1-100 fold, e.g., by about: 1-75, 1-50, 1-25, 1-20, 1-15, 1-10, 1-8, 1-6, 1-5, 1-4, 1-3 or 1-2 fold.

In some embodiments, the effective amount is sufficient to prevent death of a subject thereby reducing the cancer (e.g., tumor) death rate. In certain embodiments, the reduction in cancer (e.g., tumor) death rate is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In particular embodiments, the reduction in cancer (e.g., tumor) death rate is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

In some embodiments, the effective amount is sufficient to modulate tumor autophagy, for example, by increasing at least one tumor-inhibiting function of autophagy and/or reducing at least one tumor-promoting function of autophagy.

In some embodiments, the effective amount is sufficient to reduce cancer cell proliferation or tumor growth in the subject. In some embodiments, the reduction in cancer cell proliferation or tumor growth is by at least about 10%, e.g., by at least about: 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98% or 99%. In some embodiments, the reduction in cancer cell proliferation or tumor growth is about 10-99%, e.g., about: 10-98%, 15-98%, 15-97%, 20-97%, 20-96%, 25-96%, 25-95%, 30-95%, 30-94%, 35-94%, 35-93%, 40-93%, 40-92%, 45-92%, 45-91%, 50-91%, 50-90%, 55-90%, 55-85%, 60-85%, 60-80%, 65-80%, 65-75%, or 70-75%.

In certain embodiments, the effective amount is sufficient to modulate expression of the target protein in an immune cell.

In some embodiments, the effective amount is sufficient to modulate the subject's immune system against cancer (e.g., modulate, such as increase, an immune response to the cancer in a subject). In certain embodiments, the effective amount is sufficient to modulate (e.g., increase or decrease) at least one immune cell-related readout. Non-limiting examples of immune cell-related readouts include immune-cell activation, degranulation, maturation, migration, polarization, proliferation and recruitment; lymph-node activation, differentiation, egress, homing, innervation and polarization; cytokine production; antigen presentation; antibody-dependent cellular cytotoxicity (ADCC); and antibody-dependent cellular phagocytosis (ADCP).

In some embodiments, the effective amount is sufficient to modulate development of high endothelial venules (HEVs) and/or tertiary lymphoid organs (TLOs); immune-cell activation, degranulation, differentiation, maturation, migration, polarization, proliferation, and/or recruitment; lymph-node activation, egress, homing, and/or polarization; cytokine production; antigen presentation; inflammation; auto-antibody levels; organ function; rate and/or number of relapses and/or flare-ups; viral load; infection, or a combination of the foregoing.

In particular embodiments, the effective amount is sufficient to inhibit cancer cell growth, proliferation, metastasis, invasion or migration, or a combination of the foregoing; promote cancer cell death; induce cancer cell autophagy, or a combination of the foregoing.

In some embodiments, the effective amount is sufficient to modulate (e.g., increase or decrease) the development of HEVs and/or TLOs, migration of an immune cell (e.g., an antigen presenting cell (such as a dendritic cell and/or a macrophage) and/or a T cell), proliferation of an immune cell, recruitment of an immune cell (e.g., an antigen presenting cell (such as a dendritic cell and/or a macrophage), a monocyte, a T cell, and/or a B cell), lymph node homing of an immune cell (e.g., a dendritic cell and/or a T cell), lymph node egress of an immune cell (e.g., a dendritic cell and/or a T cell), tumor homing of an immune cell (e.g. a T cell); increase tumor egress of an immune cell (e.g., a regulatory T cell) decrease tumor egress of an immune cell (e.g., a CD8⁺ T cell) or a combination of the foregoing.

In some embodiments, the effective amount is sufficient to modulate the body's response to cancer, for example, by inhibiting the growth of cancer, reducing malignancy of cancer, inhibiting metastasis of cancer, promoting remission, modulating (increasing and/or decreasing) immune-mediated responses related to cancer, or a combination of the foregoing.

In some embodiments, the effective amount is sufficient to modulate nuclear factor kappa B (NF-κB) signaling, growth-factor signaling, cell death (e.g., apoptosis), cell cycle (e.g., mitosis), cell migration, inflammation, or a combination of the foregoing.

A therapeutic agent described herein can be administered via a variety of routes of administration, including, for example, oral, dietary, topical, transdermal, rectal, parenteral (e.g., intra-arterial, intravenous, intramuscular, subcutaneous injection, intradermal injection), intravenous infusion and inhalation (e.g., intrabronchial, intranasal or oral inhalation, intranasal drops) routes of administration, depending on the compound and the particular disease or condition to be treated. Administration can be local or systemic as indicated. The preferred mode of administration can vary depending on the particular compound chosen.

In some embodiments, the method further comprises administering a therapeutically effective amount of one or more additional therapeutic agents (e.g., a second therapeutic agent) to the subject.

Administration of two or more therapeutic agents encompasses co-administration of the therapeutic agents in a substantially simultaneous manner, such as in a pharmaceutical combination. Alternatively, such administration encompasses co-administration in multiple containers, or separate containers (e.g., capsules, powders, and liquids) for each therapeutic agent. Such administration also encompasses use of the therapeutic agents in a sequential manner, either at approximately the same time or at different times. When two or more therapeutic agents are administered, the therapeutic agents can be administered via the same administration route or via different administration routes.

In another aspect, the disclosure provides a method of modulating the expression or activity of a target protein identified in the Sequence Listing, Table A, or a variant thereof in a cell, comprising contacting the cell with an agent disclosed herein or a pharmaceutical composition disclosed herein. In some embodiments, the cell is in a subject.

In another aspect, the disclosure provides a method of identifying an agent that modulates the expression and/or activity of a target protein (e.g., a target protein in the Sequence Listing, Table A, or a variant thereof), comprising:
a) contacting a sample (e.g., a biological sample, such as cells or a tissue) comprising the target protein with an agent (e.g., a candidate agent to be tested for its ability to modulate expression and/or activity of the target); and
b) determining whether the agent modulates the expression or activity of the target protein,
wherein a difference in the expression or activity of the target protein that has been contacted with the agent compared to a reference indicates that the agent modulates the expression or activity of the target protein.

In some embodiments, a difference of at least about 10% in the expression or activity of the protein that has been contacted with the agent compared to the reference indicates that the agent modulates the expression or activity of the protein. In some embodiments, the difference is at least about: 15%, 20%, 25%, 30%, 35%, 40%, 45% or 50% or more.

In some embodiments, a decrease in the expression or activity of the target protein that has been contacted with the agent compared to the reference indicates the agent inhibits the expression or activity of the target protein. In some embodiments, an increase in the expression or activity of the protein compared to the reference indicates the agent activates the expression or activity of the protein.

### Cancer

A wide variety of cancers is treatable according to the methods described herein. In some embodiments, the cancer comprises a solid tumor (*e.g.,* a tumor of the breast, lung, prostate, colon, bladder, ovary, kidney, stomach, colon, rectum, testes, head and/or neck, pancreas, brain, skin). Accordingly, in some embodiments, the cancer is a solid tumor cancer. Solid tumor cancers that can be treated according to the methods described herein include breast cancer, lung cancer, prostate cancer, colon cancer, bladder cancer, ovarian cancer, renal cancer, gastric cancer, colon cancer, rectal cancer, colorectal cancer, testicular cancer, head and neck cancer, pancreatic cancer, brain cancer and skin cancer. In some embodiments, the cancer is a hematologic cancer (e.g., leukemia, lymphoma, myeloma). Hematologic cancers that can be treated according to the methods described herein include leukemias (e.g., acute leukemias, chronic leukemias), lymphomas (e.g., B-cell lymphoma, T-cell lymphoma) and multiple myeloma.

Examples of cancers treatable according to the methods described herein include Acute Lymphoblastic Leukemia (ALL); Acute Myeloid Leukemia (AML); Adrenocortical Carcinoma; Adrenocortical Carcinoma, Childhood; AIDS-Related Cancer (e.g., Kaposi Sarcoma, AIDS-Related Lymphoma, Primary CNS Lymphoma); Anal Cancer; Appendix Cancer; Astrocytomas, Childhood; Atypical Teratoid/Rhabdoid Tumor, Childhood, Central Nervous System; Basal Cell Carcinoma of the Skin; Bile Duct Cancer; Bladder Cancer; Bladder Cancer, Childhood; Bone Cancer (including Ewing Sarcoma, Osteosarcoma and Malignant Fibrous Histiocytoma); Brain Tumors/Cancer; Breast Cancer; Burkitt Lymphoma; Carcinoid Tumor (Gastrointestinal); Carcinoid Tumor, Childhood; Cardiac (Heart) Tumors, Childhood; Embryonal Tumors, Childhood; Germ Cell Tumor, Childhood; Primary CNS Lymphoma; Cervical Cancer; Childhood Cervical Cancer; Cholangiocarcinoma; Chordoma, Childhood; Chronic Lymphocytic Leukemia (CLL); Chronic Myelogenous Leukemia (CML); Chronic Myeloproliferative Neoplasms; Colorectal Cancer; Childhood Colorectal Cancer; Craniopharyngioma, Childhood; Cutaneous T-Cell Lymphoma (e.g., Mycosis Fungoides and Sézary Syndrome); Ductal Carcinoma In Situ (DCIS); Embryonal Tumors, Central Nervous System, Childhood; Endometrial Cancer (Uterine Cancer); Ependymoma, Childhood; Esophageal Cancer; Childhood Esophageal Cancer; Esthesioneuroblastoma; Ewing Sarcoma; Extracranial Germ Cell Tumor, Childhood; Extragonadal Germ Cell Tumor; Eye (Ocular) Cancer; Childhood Intraocular Melanoma; Intraocular Melanoma; Retinoblastoma; Fallopian Tube Cancer; Fibrous Histiocytoma of Bone, Malignant, and Osteosarcoma; Gallbladder Cancer; Gastric (Stomach) Cancer; Childhood Gastric (Stomach) Cancer; Gastrointestinal Carcinoid Tumor; Gastrointestinal Stromal Tumors (GIST); Childhood Gastrointestinal Stromal Tumors; Germ Cell Tumors; Childhood Central Nervous System Germ Cell Tumors (e.g., Childhood Extracranial Germ Cell Tumors, Extragonadal Germ Cell Tumors, Ovarian Germ Cell Tumors, Testicular Cancer); Gestational Trophoblastic Disease; Hairy Cell Leukemia; Head and Neck Cancer; Heart Tumors, Childhood; Hepatocellular (Liver) Cancer; Histiocytosis, Langerhans Cell; Hodgkin Lymphoma; Hypopharyngeal Cancer; Intraocular Melanoma; Childhood Intraocular Melanoma; Islet Cell Tumors, Pancreatic Neuroendocrine Tumors; Kaposi Sarcoma; Kidney (Renal Cell) Cancer; Langerhans Cell Histiocytosis; Laryngeal Cancer; Leukemia; Lip and Oral Cavity Cancer; Liver Cancer; Lung Cancer (Non-Small Cell and Small Cell); Childhood Lung Cancer; Lymphoma; Male Breast Cancer; Malignant Fibrous Histiocytoma of Bone and Osteosarcoma; Melanoma; Childhood Melanoma; Melanoma, Intraocular (Eye); Childhood Intraocular Melanoma; Merkel Cell Carcinoma; Mesothelioma, Malignant; Childhood Mesothelioma; Metastatic Cancer; Metastatic Squamous Neck Cancer with Occult Primary; Midline Tract Carcinoma With NUT Gene Changes; Mouth Cancer; Multiple Endocrine Neoplasia Syndromes; Multiple Myeloma/Plasma Cell Neoplasms; Mycosis Fungoides; Myelodysplastic Syndromes, Myelodysplastic/Myeloproliferative Neoplasms; Myelogenous Leukemia, Chronic (CML); Myeloid Leukemia, Acute (AML); Myeloproliferative Neoplasms, Chronic; Nasal Cavity and Paranasal Sinus Cancer; Nasopharyngeal Cancer; Neuroblastoma; Non-Hodgkin Lymphoma; Non-Small Cell Lung Cancer; Oral Cancer, Lip and Oral Cavity Cancer and Oropharyngeal Cancer; Osteosarcoma and Malignant Fibrous Histiocytoma of Bone; Ovarian Cancer; Childhood Ovarian Cancer; Pancreatic Cancer; Childhood Pancreatic Cancer; Pancreatic Neuroendocrine Tumors; Papillomatosis (Childhood Laryngeal); Paraganglioma; Childhood Paraganglioma; Paranasal Sinus and Nasal Cavity Cancer; Parathyroid Cancer; Penile Cancer; Pharyngeal Cancer; Pheochromocytoma; Childhood Pheochromocytoma; Pituitary Tumor; Plasma Cell Neoplasm/Multiple Myeloma; Pleuropulmonary Blastoma; Pregnancy and Breast Cancer; Primary Central Nervous System (CNS) Lymphoma; Primary Peritoneal Cancer; Prostate Cancer; Rectal Cancer; Recurrent Cancer; Renal Cell (Kidney) Cancer; Retinoblastoma; Rhabdomyosarcoma, Childhood; Salivary Gland Cancer; Sarcoma (e.g., Childhood Rhabdomyosarcoma, Childhood Vascular Tumors, Ewing Sarcoma, Kaposi Sarcoma, Osteosarcoma (Bone Cancer), Soft Tissue Sarcoma, Uterine Sarcoma); Sézary Syndrome; Skin Cancer; Childhood Skin Cancer; Small Cell Lung Cancer; Small Intestine Cancer; Soft Tissue Sarcoma; Squamous Cell Carcinoma of the Skin; Squamous Neck Cancer with Occult Primary, Metastatic; Stomach (Gastric) Cancer; Childhood Stomach (Gastric) Cancer; T-Cell Lymphoma, Cutaneous (e.g., Mycosis Fungoides and Sèzary Syndrome); Testicular Cancer; Childhood Testicular Cancer; Throat Cancer (e.g., Nasopharyngeal Cancer, Oropharyngeal Cancer, Hypopharyngeal Cancer); Thymoma and Thymic Carcinoma; Thyroid Cancer; Transitional Cell Cancer of the Renal Pelvis and Ureter; Ureter and Renal Pelvis, Transitional Cell Cancer; Urethral Cancer; Uterine Cancer, Endometrial; Uterine Sarcoma; Vaginal Cancer; Childhood Vaginal Cancer; Vascular Tumors; Vulvar Cancer; and Wilms Tumor and Other Childhood Kidney Tumors.

Metastases of the aforementioned cancers can also be treated in accordance with the methods described herein. In some embodiments, the cancer is a metastatic cancer.

In some embodiments, the cancer is selected from lung cancer, breast cancer, Hodgkin's lymphoma, diffuse large B-cell lymphoma, follicular lymphoma, primary central nervous system lymphoma, chronic lymphocytic leukemia, epithelial ovarian cancer, prostate cancer, squamous cell carcinoma, non-melanoma skin cancer, nasal polyps, basal cell carcinoma, keratinocyte cancer, multiple myeloma, serous invasive ovarian cancer, hepatocellular carcinoma, small cell lung carcinoma, adenocarcinoma, lung adenocarcinoma, non-small cell lung cancer, ovarian cancer or colorectal cancer.

Unless otherwise defined, all terms of art, notations and other scientific terms or terminology used herein are intended to have the meanings commonly understood by those of skill in the art to which this disclosure pertains. In some cases, terms with commonly understood meanings are defined herein for clarity and/or for ready reference, and the inclusion of such definitions herein should not necessarily be construed to represent a substantial difference over what is generally understood in the art. It will be further understood that terms, such as those defined in commonly-used dictionaries, should be interpreted as having a meaning that is consistent with their meaning in the context of the relevant art and/or as otherwise defined herein.

The terminology used herein is for the purpose of describing particular embodiments only and is not intended to be limiting.

As used herein, the indefinite articles "a," "an" and "the" should be understood to include plural reference unless the context clearly indicates otherwise.

Throughout this specification and the claims which follow, unless the context requires otherwise, the word "comprise," and variations such as "comprises" and "comprising", will be understood to imply the inclusion of, *e.g.,* a stated integer or step or group of integers or steps, but not the exclusion of any other integer or step or group of integer or step. When used herein, the term "comprising" can be substituted with the term "containing" or "including."

As used herein, "consisting of" excludes any element, step, or ingredient not specified in the claim element. When used herein, "consisting essentially of" does not exclude materials or steps that do not materially affect the basic and novel characteristics of the claim. Any of the terms "comprising," "containing," "including," and "having," whenever used herein in the context of an aspect or embodiment of the disclosure, can in some embodiments, be replaced with the term "consisting of," or "consisting essentially of" to vary scopes of the disclosure.

As used herein, the conjunctive term "and/or" between multiple recited elements is understood as encompassing both individual and combined options. For instance, where two elements are conjoined by "and/or," a first option refers to the applicability of the first element without the second. A second option refers to the applicability of the second element without the first. A third option refers to the applicability of the first and second elements together. Any one of these options is understood to fall within the meaning, and, therefore, satisfy the requirement of the term "and/or" as used herein. Concurrent applicability of more than one of the options is also understood to fall within the meaning, and, therefore, satisfy the requirement of the term "and/or."

When a list is presented, unless stated otherwise, it is to be understood that each individual element of that list, and every combination of that list, is a separate embodiment. For example, a list of embodiments presented as "A, B, or C" is to be interpreted as including the embodiments, "A," "B," "C," "A or B," "A or C," "B or C," or "A, B, or C."

### Exemplification

### Example 1: Validation of a Target Protein as an oncogene in a cell cycle assay

This example demonstrates the validation of a target protein of the disclosure as a contributor to tumor growth and/or accelerated cell cycle progression.

Stable tumor cell lines with increased or decreased levels of the target protein or controls are generated. The proliferation rates of the generated cell lines are compared. If the proliferation rate of the generated cell lines correlates with the level of target protein then the target protein is an oncogene.

### Materials and Methods:

*Over-expression:* Stable over-expression tumor cell lines are made by transfection using lipofectamine 3000 (ThermoFisher) of pcDNA3.1_Myc plasmids (GenScript Inc) containing: 1) no insert, 2) a target protein-encoding sequence, 3) a scrambled version of the target protein-encoding sequence (negative control), or 4) the Gfi-1 protein (positive control for cell proliferation; doi.org/10.1038/sj.onc.1205216), into Jurkat E6-1 (T-cell line; ATCC). This vector adds a Myc-tag (EQKLISEEDL) to the C-terminus of the expressed protein. Post-transfection, stable cell mixed populations are selected for with neomycin for 6-10 days. Expression of the target protein and scramble protein are confirmed by immunoblot of 10 µg of cell lysate using an anti-myc-tag polyclonal antibody (Abcam). Clonal cell lines are produce by serial dilutions with continuous selection.

*Target Protein Knock-out:* The effects of target protein deletion in a disease relevant tumor cell line (Jurkat E6-1) are determined by Alt-R CRISPR-Cas9 knock-out via RNP particle transfected via electroporation (Idtdna.com). The target protein is knocked-out as well as the REL protein (positive control; • DOI: 10.1016/j.molimm.2020.06.029) and a no-guide (negative control). Neomycin resistance is conferred on the successfully transfected cells. Clonal cell lines are produced by serial dilutions with continuous selection. Knock-out is confirmed by genotype sequencing.

*Proliferation:* Clonal cell lines that over-express the target protein or control, wild-type Jurkat E6-1 cells, and clonal cell lines with the endogenous target protein and controls knocked out are plated at 10,000 or 40,000 cells per well, grown in standard media and then subjected to the MTT (3-(4,5-dimethylthiazol-2-yl)-2,5-diphenyltetrazolium bromide) assay. The MTT assay (Abcam) is a measure of the metabolic activity of group of cells. The higher the metabolic activity the higher the proliferation rate.

*Cell Cycle Progression:* Clonal cell lines that over-express the target protein or control, wild-type Jurkat E6-1 cells, and clonal cell lines with the endogenous target protein and controls knocked out are plated at 10,000 or 40,000 cells per well grown in both standard media and, separately, media supplemented with phorbol ester compound (at levels known to induce G1 cell cycle arrest) and then subjected to propridium iodide staining and cell cycle analysis by cytometer (doi.org/10.1038/sj.onc.1205216).

Over-expression of Gfi-1 (positive control) leads to increased Jurkat cell proliferation and accelerates S-phase entry of Jurkat cells compared to wild-type and scramble over-expression. Knock-out of the c-REL oncogene leads to decreased proliferation compared to the no guide control. Interestingly, over-expression of the target protein, like the positive control oncogenes leads to increased proliferation and accelerates S-phase entry while knock out of the target protein leads to a decrease in proliferation. Taken together, these experiments demonstrate that the target protein is an oncogene and that targeting this protein may benefit patients suffering from cancer, specifically leukemias.

### Example 2: Validation of a target protein as an oncogene wherein the target protein has a gain of function driver mutation

This example demonstrates the validation of a target protein of the disclosure that contains mutations (determined by Genome Wide Association Studies (GWAS) and/or cataloged in The Cancer Genome Atlas (TCGA)) found in cancer tissue. These mutations can facilitate cancer progression by activating an oncogenic target protein.

Stable tumor cell lines over-expressing the target protein with wild type and cataloged TCGA or GW AS mutations are generated. Furthermore, in tumor cell lines that express the wild-type target protein, CRISPR-Cas9 is used to replace the wild type protein with the mutated target protein. The proliferation rate and apoptosis rate (basal and induced) of the generated cell lines are compared. If the target protein mutation increases the proliferation rate and/or slows the apoptosis rate, then the target protein is an oncogene and the mutation is a gain-of-function mutation with respect to the cancer disease phenotype.

### Materials and Methods:

*Over-expression:* Stable over-expression tumor cell lines are made by transfection using lipofectamine 3000 (ThermoFisher) of pcDNA3.1 _Myc plasmids (GenScript Inc) containing: 1) no insert, 2) wild type target protein encoding sequence, 3) a mutant version of the target protein encoding sequence (a mutation found in cancers; TCGA and/or GWAS), or 4) the oncoprotein Gfi-1 protein (positive control for cell proliferation; doi.org/10.1038/sj.onc.1205216), into Jurkat E6-1 (T-cell line; ATCC). This vector adds a Myc-tag (EQKLISEEDL) to the C-terminus of the expressed protein. Post-transfection, stable cell mixed populations are selected for with neomycin for 6-10 days. Expression of the target protein and mutant protein are confirmed by immunoblot of 10 µg of cell lysate using anti-myc-tag polyclonal antibody (Abcam). Clonal cell lines are produce by serial dilutions with continuous selection.

*Mutant Target Protein Knock-in:* The wild-type target protein is expressed in Jurkat E6 cells. To determine if mutations seen in some cancer patients confer disease associated phenotypes on cancer cells, CRISPR-Cas9 is used to replace the wild-type target protein with the mutant versions of the target protein. As a control for a CRISPR-Cas9 treated cell line having increased apoptosis, the FAU tumor suppressor is knocked-out separately. Clonal cell lines are produced by serial dilutions with continuous selection. Knock-out and replacement is confirmed by genotype sequencing.

*Proliferation:* Clonal cell lines are plated at 10,000 or 40,000 cells per well grown in standard media, and 24 hours later are subjected to the MTT (3-(4,5-dimethylthiazol-2-yl)-2,5-diphenyltetrazolium bromide) assay. The MTT assay (Abcam) is a measure of the metabolic activity of group of cells. The higher the metabolic activity the higher the proliferation rate.

*Apoptosis:* Clonal cell lines are plated at 10,000 or 40,000 cells per well, grown either in standard media or media supplemented with an apoptosis-inducing drug such as Venetoclax and then subjected to the RealTime-Glo(tm) Annexin 5 Apoptosis and Necrosis Assay (Promega).

Over-expression of GFI-1 oncoprotein (positive control) leads to increased Jurkat cell proliferation to wild-type and scramble over-expression. Knock-out of the FAU tumor suppressor also leads to increased proliferation and decrease in apoptosis compared to the no guide control. Interestingly, both over-expression of the mutant target protein and replacement of the wild-type target protein with mutant target protein, compared to wild-type Jurkat, lead to increased proliferation and/or a decrease in apoptosis. Taken together, these experiments may explain why the mutations observed in cancer patient GWAS/TCGA studies occur; as they lead to a selective advantage over other tumor cells in that they exhibit increased proliferation and decreased apoptosis.

### Example 3: Validation of a target protein as a tumor suppressor in an apoptosis assay

This example demonstrates the validation of a target protein of the disclosure as a tumor suppressor, specifically, a protein that induces apoptosis in tumor cells.

Stable tumor cell lines with increased or decreased levels of the target protein or controls are generated. The proliferation rate and apoptosis rate (basal and induced) of the generated cell lines are compared. If the proliferation rate slows and/or the apoptosis rate increases in the generated cell lines with an increase in target protein expression, then the target protein is a tumor suppressor and gain of function delivery of the target protein may provide a benefit in some types of cancer.

### Materials and Methods:

*Over-expression:* Stable over-expression tumor cell lines are made by transfection using lipofectamine 3000 (ThermoFisher) of pcDNA3.1 _Myc plasmids (GenScript Inc) containing: 1) no insert, 2) target protein encoding sequence, 3) a scrambled version of the target protein encoding sequence (negative control), or 4) the Fau protein (positive control for cell proliferation; doi:10.1016/j.bbadis.2011.04.009), into Jurkat E6-1 (T-cell line; ATCC). This vector adds a Myc-tag (EQKLISEEDL) (SEQ ID NO: 39018) to the C-terminus of the expressed protein. Post-transfection, stable cell mixed populations are selected for with neomycin for 6-10 days. Expression of the target protein and scramble protein are confirmed by immunoblot of 10 µg of cell lysate using anti-myc-tag polyclonal antibody (Abcam). Clonal cell lines are produced by serial dilutions with continuous selection.

*Target Protein Knock-out:* The effects of target protein deletion in a disease relevant tumor cell line (Jurkat E6-1) are determined by Alt-R CRISPR-Cas9 knock-out via RNP particle transfected via electroporation (Idtdna.com). The target protein is knocked-out as well as the SODD protein (positive control; • DOI dx.doi.org/10.4238/2014.March.24.6) and a no-guide (negative control). Neomycin resistance is conferred on successfully transfected cells. Clonal cell lines are produced by serial dilutions with continuous selection. Knock-out is confirmed by genotype sequencing.

*Proliferation:* Clonal cell lines that over-express the target protein or control, wild-type Jurkat E6-1 cells, and clonal cell lines with the endogenous target protein and controls knocked out are plated at 10,000 or 40,000 cells per well, grown in standard media, and then subjected to the MTT (3-(4,5-dimethylthiazol-2-yl)-2,5-diphenyltetrazolium bromide) assay. The MTT assay (Abcam) is a measure of the metabolic activity of group of cells. The higher the metabolic activity the higher the proliferation rate.

*Apoptosis:* Clonal cell lines that over-express the target protein or control, wild-type Jurkat E6-1 cells, and clonal cell lines with the endogenous target protein and controls knocked out are plated at 10,000 or 40,000 cells per well, grown either in standard media or media supplemented with an apoptosis-inducing drug such as Venetoclax and then subjected to the RealTime-Glo(tm) Annexin 5 Apoptosis and Necrosis Assay (Promega).

*Mouse Xenograft Model:* Over-expression and knock out stable cell lines produced above are used in a mouse xenograft model to determine the in vivo effect on tumor growth.

Results: Over-expression of FAU tumor suppressor (positive control) leads to decreased Jurkat cell proliferation and increased basal apoptosis levels compared to wild-type and scramble over-expression. Knock-out of the SODD also leads to decreased proliferation and increase in apoptosis compared to the no guide control. Interestingly, over-expression of the target protein, like the FAU positive control tumor suppressor leads to decreased proliferation and increased apoptosis while knock out of the target protein leads to an increase in proliferation and a decrease in apoptosis. These in vitro changes translate to decreases in tumor growth rate in a mouse xenograft model. Taken together, these experiments demonstrate that the target protein is a tumor suppressor and that delivering this protein to a tumor may provide a benefit to patients suffering from cancer.

### Example 4: Validation of a target protein as a tumor suppressor wherein the protein has a loss of function mutation

This example demonstrates the validation of a target protein of the disclosure as a tumor suppressor. Specifically, a protein that has exhibited a loss-of-function mutation in cancer (determined by Genome Wide Association Studies (GWAS) and/or cataloged in The Cancer Genome Atlas (TCGA)) that increase tumor cell proliferation and/or apoptosis by inactivating the target protein.

Stable tumor cell lines over-expressing the target protein with wild type or one or more cataloged TCGA or GWAS mutations are generated. Furthermore, in tumor cell lines that express the wild-type target protein, CRISPR-Cas9 is used to replace the wild type protein with the mutated target protein. The proliferation rate and apoptosis rate (basal and induced) of the generated cell lines are compared. If the target protein over-expression decreases proliferation and/or increases apoptosis, and the over-expression of the mutated target protein shows no such effects, then the target protein is a tumor suppressor and the cancer-associated mutation is a loss-of-function mutation. Likewise, if the replacement of the wild type allele with the cancer-associated mutation by CRISPR-Cas9 increases proliferation and/or decreases apoptosis, then the target protein is a tumor suppressor.

### Materials and Methods:

*Over-expression:* Stable over-expression tumor cell lines are made by transfection using lipofectamine 3000 (ThermoFisher) of pcDNA3.1 _Myc plasmids (GenScript Inc) containing: 1) no insert, 2) wild type target protein encoding sequence, 3) a mutant version of the target protein encoding sequence (a mutation found in cancers; TCGA and/or GWAS), or 4) the tumor suppressor protein FAU (positive control for decreased cell proliferation/increased apoptosis), into Jurkat E6-1 (T-cell line; ATCC). This vector adds a Myc-tag (EQKLISEEDL SEQ ID NO: 39018) to the C-terminus of the expressed protein. Post-transfection, stable cell mixed populations are selected for with neomycin for 6-10 days. Expression of the target protein and mutant protein are confirmed by immunoblot of 10 µg of cell lysate using anti-myc-tag polyclonal antibody (Abcam). Clonal cell lines are produce by serial dilutions with continuous selection.

*Mutant Target Protein Knock-in:* The wild-type target protein is expressed in Jurkat E6 cells. To determine if mutations seen in some cancer patients confer disease associated phenotypes on cancer cells, CRISPR-Cas9 is used to replace the wild-type target protein with the mutant versions of the target protein. As a control the FAU tumor suppressor protein is knocked-out which should lead an increase in proliferation and/or decrease in apoptosis. Clonal cell lines are produced by serial dilutions with continuous selection. Knock-out and replacement is confirmed by genotype sequencing.

*Proliferation:* Clonal cell lines are plated at 10,000 or 40,000 cells per well, grown in standard media, and 24 hours later are subjected to the MTT (3-(4,5-dimethylthiazol-2-yl)-2,5-diphenyltetrazolium bromide) assay. The MTT assay (Abcam) is a measure of the metabolic activity of group of cells. The higher the metabolic activity the higher the proliferation rate.

*Apoptosis:* Clonal cell lines are plated at 10,000 or 40,000 cells per well, grown either in standard media or media supplemented with an apoptosis-inducing drug such as Venetoclax and then subjected to the RealTime-Glo(tm) Annexin 5 Apoptosis and Necrosis Assay (Promega).

Results: Over-expression of the FAU tumor suppressor protein (positive control) leads to a decrease in Jurkat cell proliferation and/or an increase in apoptosis compared to wild-type and scramble over-expression. Knock-out of the FAU tumor suppressor leads to increased proliferation and decrease in apoptosis compared to the no guide control. Interestingly, over-expression of the target protein but not cancer-associated mutant target protein leads to a decrease in Jurkat cell proliferation and/or apoptosis. Likewise, replacement of the target protein with cancer-associated mutant target protein leads to an increase in proliferation and/or a decrease in apoptosis. Taken together, these experiments may explain why the mutations observed in cancer patient GWAS/TCGA studies occur; as they lead to a selective advantage over other tumor cells in that they exhibit increased proliferation and decreased apoptosis.

### Example 5: Validation of a target protein as an oncogene or tumor suppressor by cancer growth and proliferation

This example demonstrates the ability of a target protein of the disclosure to function as an oncogene or tumor suppressor in a cancer cell. In this example, a library of lentiviral-encoded guide RNAs (gRNA) that individually target proteins of interest genes is synthesized (Cellecta, Inc.) to obtain a library coverage of six unique gRNAs per gene.

### Materials and Methods:

Cancer cell lines (dtp.cancer.gov/discovery_development/nci-60/cell_list.htm) are transduced with a lentiviral-encoded Cas9 nuclease at a high multiplicity of infection (MOI), then transduced with the gRNA lentivirus library at a low MOI of 0.5 to ensure that individual cells received approximately one gRNA. The gRNA library vector also encodes puromycin resistance. One-day post-transduction, cells are incubated with puromycin for four days to select for successfully-transduced cells.

Following transduction and selection, 10 x 10⁶ cells are harvested to serve as the "baseline" population from which the growth effects of individual genes can be compared. 10 x 10⁶ cells are plated in manufacturer's recommended medium and split twice weekly for four weeks, by re-plating 10 x 10⁶ cells at each split.

Following the four weeks of cell/tumor growth, DNA from in vitro samples is isolated and lysedis using a DNA extraction kit (Qiagen DNeasy Blood and Tissue Kit), and concentrated by ethanol precipitation. The DNA samples are amplified by two rounds of PCR using manufacturer's recommended primers and analyzed by next-generation sequencing (BGIAmerica).

Sequencing results are analyzed to call hits using a Model-based Analysis of Genome-wide CRISPR-Cas9 Knockout (MAGeCK) algorithm as described by Li W et al., Genome Biology 2014 and Li W et al., Genome Biology 2015. In brief, sequencing reads are normalized to their medians, the variance of read counts for individual gRNAs are estimated and normalized, and individual gRNA read count differences are ranked against each other. Target genes are called based on whether multiple gRNAs targeting a single gene ranked near the top of the gRNA ranking list. gRNAs that are observed in the analysis are indicative of its corresponding target proteins acting as an oncogene.

Target genes are quantified along three parameters: the Beta score, essentially the magnitude of the effect (log-fold change in gRNA count); the p-value; and the false discovery rate (FDR). Beta scores <0 indicated that the six gRNAs targeting a single gene are absent from the late-stage sample compared to the baseline sample, and are a good indication that the gene is "dropping out" in the course of tumor growth. P-value and FDR both reflected the confidence that the result is not artifactual, with a lower value indicating higher confidence. Thresholds for calling hits are P-value <0.1 and FDR <0.5. gRNAs that are no longer observed in the analysis (dropout) are indicative of its corresponding target proteins acting as a tumor suppressor.

### Example 6: Validation of a target protein as a tumor suppressor in an apoptosis assay

This example demonstrates the validation of a target protein of the disclosure, SEQ ID NO: 37997, as a secreted tumor suppressor, and a potential target for the treatment of multiple cancers. Multiple tumor cell lines were treated with synthetically produced SEQ ID NO: 37997 protein. Twenty-four hours later basal levels of apoptosis were measured.

### Materials and Methods:

*Cell Culture:* Tumor cell lines HCT-116 (Colon Cancer Cell line, ATCC) and U2OS (Osteoscarcoma Cell line, ATCC) cells were grown adherently in ATCC-formulated McCoy's 5a medium supplemented with 10% FBS and 1X penicillin-streptomycin at 37°C (5% CO2). Jurkat cells (acute T-cell leukemia cell line, ATCC) were grown as suspension cultures in ATCC-formulated RPMI-1640 supplemented with 10% FBS and 1X penicillin-streptomycin at 37°C (5% CO2). HEP-G2 cells (Liver Cancer Cell line, ATCC) cells were grown adherently in ATCC-formulated Eagle's Minimum Essential Medium (EMEM) supplemented with 10% FBS and 1X penicillin-streptomycin at 37°C (5% CO2).

*Peptide Treatment:* Target protein SEQ ID NO: 37997 was synthesized through Fluorenylmethyloxycarbonyl (Fmoc) protection group chemistry using Solid Phase Peptide Synthesis (SPPS). 5µM of target peptide and control peptides were added to a 96 well plate containing 10,000 cells (100µL/well). A white-walled 96-well plate adequate for cell culture and compatible with the luminometer was used. (Corning)

*Apoptosis Assay:* Following 24hrs of peptide treatment, an equal volume reconstituted Caspase-Glo^{®} 3/7 assay reagent mixture (Promega) was added to the cells. The contents of wells were gently mixed using a plate shaker at 300-500rpm for 30 seconds. The plate was then Incubate at room temperature for 3 hours. The treated plates were then read for luminescence on Promega^{™} GloMax^{®} Plate Reader. A blank reaction that contained caspase-Glo^{®} 3/7 reagent, vehicle and cell culture medium without cells was used to measure background luminescence. The blank reaction values were subtracted from experimental values. A negative control that contained caspase-Glo^{®} 3/7 reagent and vehicle-treated cells in medium was included to determine the basal caspase activity of the cell culture system. Significant difference in apoptosis was determined using ANOVA analysis by comparing cells treated with SEQ ID NO: 37997 peptide to cells treated with an irrelevant control peptide, or vehicle control treated cells.

### Results:

Treatment of multiple cell lines with SEQ ID NO: 37997 peptide led to an increase in basal apoptosis levels. SEQ ID NO: 37997 is a tumor suppressor and is suitable for the treatment of multiple cancers (FIG. 1, FIG. 2, and Table C).

**Table C. List of ORF peptides inducing significant apoptosis in multiple cell lines. Peptides with z-score > 2 compared to vehicle control were considered as significant.**

| HCT-116 | Jurkat |
|---|---|
| SEQ ID NO: 29485 | SEQ ID NO: 34296 |
| SEQ ID NO: 27000 | SEQ ID NO: 34224 |
| SEQ ID NO: 27193 | SEQ ID NO: 37407 |
| HepG2 | SEQ ID NO: 34229 |
| SEQ ID NO: 34224 | SEQ ID NO: 36828 |
| SEQ ID NO: 26966 | SEQ ID NO: 33599 |
| SEQ ID NO: 39056 | SEQ ID NO: 29485 |
| U2OS | SEQ ID NO: 27000 |
| SEQ ID NO: 34229 | SEQ ID NO: 33579 |
| SEQ ID NO: 36828 | SEQ ID NO: 35814 |
| SEQ ID NO: 38076 | SEQ ID NO: 34508 |
| SEQ ID NO: 33599 | SEQ ID NO: 39056 |
| SEQ ID NO: 27000 | SEQ ID NO: 27193 |
| SEQ ID NO: 27193 | |

### Example 7: Validation of a target protein as a tumor suppressor wherein the protein has a loss of function mutation

This example demonstrates the validation of target proteins SEQ ID NO: 27301 and SEQ ID NO: 30462, as contributors to tumor growth. Stable tumor cell lines with increased levels of each target protein or controls were generated. The proliferation rate of the generated cell lines were compared. If the proliferation rate of the generated cell lines was increased with increased level of target protein, then the target protein is an oncogene.

Stable tumor cell lines over-expressing the target protein with wild type or one or more cataloged TCGA or GWAS mutations are generated. Furthermore, in tumor cell lines that express the wild-type target protein, CRISPR-Cas9 is used to replace the wild type protein with the mutated target protein. The proliferation rate and apoptosis rate (basal and induced) of the generated cell lines are compared. If the target protein over-expression decreases proliferation and/or increases apoptosis, and the over-expression of the mutated target protein shows no such effects, then the target protein is a tumor suppressor and the cancer-associated mutation is a loss-of-function mutation. Likewise, if the replacement of the wild type allele with the cancer-associated mutation by CRISPR-Cas9 increases proliferation and/or decreases apoptosis, then the target protein is a tumor suppressor.

### Materials and Methods:

*Over-expression:* Stable over-expression tumor cell lines were made by transduction using lentivirus. For virus production, HEK-293T cells were cultured in DMEM with high glucose and GlutaMAX^{™} medium (ThermoFisher) supplemented with 10% FBS. After 24h, cells were transfected using lipofectamine 3000 (ThermoFisher) of Lentiviral packaging plasmid mix (CELLECTA) and pGenLenti plasmids (GenScript Inc) containing: 1) no insert, 2) Target Protein encoding sequence, and 3) the AP2A1 protein (positive control for cell proliferation; https://doi.org/10.1038/sj.onc.1205216), into HCT116 (Colon cancer cell line; ATCC). A Myc-tag (EQKLISEEDL) was added to the c-terminus of the expressed protein. After 48hr post-transfection, culture media containing viruses was collected, loaded onto a 10ml syringe and filtered through a 0.45 µm filter. For cell transduction, HCT116 colorectal cancer cells were seeded in 6-well plates, the virus was added to the cells. After post-transduction, stable cell mixed populations are selected for with puromycin for 14 days. Expression of the Target Protein and controls are confirmed by immunoblot of 50 ug of cell lysate using anti-myc-tag polyclonal antibody (CST).

*Western Blot:* 50 ug of cell lysate from control and Target Protein stable cell lines were separated by NuPAGE 4-12% Bis-Tris Gel (invitrogen), transferred to PVDF membrane (Thermofisher) and subjected to anti-Myc-tag (CST) and anti-GAPDH (CST) primary antibodies followed by anti-Rabbit HRP secondary antibodies (CST). The HRP signal is visualized with the iBright system.

*Proliferation:* Polyclonal cell lines that over-express the Target Protein or control, wild-type HCT-116 cells, were plated in 96 well plate (10,000 or 5,000 cells per well) grown in standard media in both low serum (0.1% FBS) and high serum (10% FBS) conditions, respectively. After 48 hours, the cells are subjected to the WST-1 (3-(4,5-dimethylthiazol-2-yl)-2,5-diphenyltetrazolium bromide) assay. The WST-1 assay (Sigma) is a measure of the metabolic activity of group of cells. The higher the metabolic activity the higher the proliferation rate. Significant differences in proliferation are determined by comparing protein-expressing cell lines with a stable cell line of the empty expression vector (control) using ANOVA analysis.

*Maestro Z Impedance-based assay:* The Maestro Z platform (Axion Biosystems) uses impedance measurements (ohms, Ω) to quantify the presence of cells on the electrode. Since impedance is noninvasive and label-free, impedance assay is used to quantify dynamic cellular growth over time. For impedance recording, cells were seeded in 96 well assay plate, and the plate was docked in the Maestro Z and the automatic environment controls set the chamber to 37°C and 5% CO2. Impedance measurements were recorded every minute for the 2 days.

### Results:

Over-expression of AP2A (positive control oncogene) leads to increased HCT-116 cell proliferation compared to control cells. Interestingly, over-expression of the target protein, like the positive control oncogenes leads to increased proliferation. These experiments demonstrate that the target protein is an oncogene and that targeting this protein could have benefits to patients suffering from cancer such as colon cancer (FIG. 3, FIG. 4, FIG. 5, Table D).

**Table D.**

| | |
|---|---|
| SEQ ID NO: 33586 | *** |
| SEQ ID NO: 36829 | ** |
| SEQ ID NO: 38556 | ** |

### Example 8: Validation of a target protein as an oncogene or tumor suppressor by cancer growth and proliferation

*Proliferation:* HCT-116 cells were plated in 96 well plate (5,000 cells/well) grown in standard media supplemented with 10% FBS. After 48 hours incubation at 37°C (5% CO₂), the cells are subjected to the WST-1 (3-(4,5-dimethylthiazol-2-yl)-2,5-diphenyltetrazolium bromide) assay. The WST-1 assay (Sigma) is a measure of the metabolic activity (i.e. proliferative rate) of cells. Significant differences in proliferation are determined by comparing cells treated with 10 uM SEQ ID NO: 37997 protein to cells treated with 10 uM of an irrelevant control peptide, or untreated cells using ANOVA analysis (FIG. 6, Table E).

**Table E: Other Hits from high sera and low sera experiments. In blue: tumor suppressors. In Red: tumor activators.**

| High sera | | | | | | | | |
|---|---|---|---|---|---|---|---|---|
| U2OS | HCT116 | U2OS | HCT116 | U2OS | HCT116 | HCT116 | HCT116 | |
| 10 uM | 10 uM | 5 uM | 5 uM | 1 uM | 1 uM | 0.5 uM | 0.1 uM | |
| 8 SD | 4 SD | 5 SD | 3 sd | 5 SD | 2 SD | 3 SD | 3 SD | |
| | | | | | | | | |
| -12.108362 | -5.8562373 | | | -1.710525 | -2.8670694 | | -5.0965093 | SEQ ID NO: 34508 |
| -11.845722 | -7.1767171 | | | -2.3435539 | -0.1040104 | | -0.7167673 | SEQ ID NO: 27000 |
| -10.62007 | -6.5368271 | | | 0.85526249 | 0.45900239 | | -1.0875869 | SEQ ID NO: 27000 |
| -14.680884 | -4.8432666 | | | -2.5657875 | -0.6104958 | | 0.54266292 | SEQ ID NO: 33599 |
| -6.3033519 | 0.06331067 | | | -5.7915413 | -0.8795661 | | 0.1989764 | SEQ ID NO: 29485 |
| -4.0069384 | 0.27811475 | | | -15.926738 | -1.3521351 | | -4.6759455 | SEQ ID NO: 29485 |
| -10.57293 | -8.6057295 | | | -0.8283251 | -3.6720191 | | 0.99262092 | SEQ ID NO: 33681 |
| -3.6702209 | -0.5064854 | | | -15.18596 | -0.4883966 | | -0.495199 | SEQ ID NO: 38081 |
| -5.7578695 | 0.27133146 | | | -5.7713382 | -0.4363914 | | 2.1593462 | SEQ ID NO: 29230 |
| -13.232998 | -11.511237 | | | -5.9599 | -5.79971 | | -3.1135285 | SEQ ID NO: 37997 |
| | | -0.8797654 | -0.0232911 | | | 0.20379745 | | SEQ ID NO: 34508 |
| | | 15.3292461 | 0.83265817 | | | 1.07915604 | | SEQ ID NO: 27193 |
| | | -6.824847 | 0.00873418 | | | 0.53375523 | | SEQ ID NO: 34047 |
| | | 1.19968013 | -1.7798311 | | | -1.4663712 | | SEQ ID NO: 28759 |
| | | -3.8123169 | -1.4566666 | | | -0.9491139 | | SEQ ID NO: 38017 |
| | | -4.1322316 | -0.2232067 | | | 0.38139238 | | SEQ ID NO: 38073 |
| | | -0.8797654 | -1.4760758 | | | -0.8520674 | | SEQ ID NO: 38076 |
| | | 3.09250877 | -1.9108437 | | | -0.6812658 | | SEQ ID NO: 26298 |
| | | -6.6915492 | -1.7012235 | | | -0.3144304 | | SEQ ID NO: 26298 |

| Low sera | | | | | | | | | | |
|---|---|---|---|---|---|---|---|---|---|---|
| U2OS | HCT116 | U2OS | HCT116 | U2OS | HCT116 | U2OS | HCT116 | U2OS | U2OS | |
| 10 uM | 10 uM | 5 uM | 5 uM | 1 uM | 1 uM | 0.5 uM | 0.5 uM | 0.1 uM | 0.1 uM | |
| 6 SD | 3 SD | 2 SD | 2 SD | 5 SD | 3 SD | 3 SD | 3 SD | 4 SD | 2 SD | |
| | | | | | | | | | | |
| - 11.737394 | - 3.6284316 | | | 4.2754317 1 | - 1.6002947 | | | 3.4747809 4 | 1.3349501 7 | SEQ ID NO: 34508 |
| -11.23299 | - 3.5893498 | | | 5.6845494 6 | - 0.0370248 | | | 7.7582090 5 | - 1.0366947 | SEQ ID NO: 27000 |
| -12.97038 | - 5.0538869 | | | 9.0312278 7 | - 0.2447751 | | | 7.5420360 4 | - 1.1889079 | SEQ ID NO: 27000 |
| - 8.3907153 | - 5.2904343 | | | 4.3234601 5 | - 0.9914422 | | | 2.0096083 3 | - 0.8885955 | SEQ ID NO: 33599 |
| 5.6124917 9 | - 0.7363824 | | | 3.7950372 5 | - 1.2650144 | | | 2.0256211 5 | 0.1542700 5 | SEQ ID NO: 29485 |
| 4.3234601 5 | - 1.0572641 | | | 2.3218582 3 | - 2.4086697 | | | 1.9215378 5 | - 2.5156303 | SEQ ID NO: 29485 |
| - 6.8614914 | - 2.5835091 | | | 9.3755034 1 | 0.1254729 7 | | | 4.9879919 9 | - 1.0099546 | SEQ ID NO: 33681 |
| - 2.3218582 | - 2.4765485 | | | 1.5212174 6 | - 3.5893498 | | | 2.081666 | - 3.4700477 | SEQ ID NO: 38081 |
| 2.6821465 8 | - 2.1618376 | | | 3.8751013 2 | -1.090175 | | | 2.0096083 3 | - 2.2955384 | SEQ ID NO: 29230 |
| - 16.092879 | - 6.9154121 | | | 0.3202563 1 | - 3.0154653 | | | 1.9215378 5 | - 2.3593033 | SEQ ID NO: 37997 |
| | | 1.3440954 64 | 1.9316341 1 | | | 5.0433323 2 | 0.9567422 6 | | | SEQ ID NO: 34508 |
| | | 2.3194570 8 | 0.6896841 3 | | | 1.9745121 8 | 1.0811965 4 | | | SEQ ID NO: 27193 |
| | | -5.958031 | 0.3240996 8 | | | 2.3313517 3 | 1.5608640 7 | | | SEQ ID NO: 34047 |
| | | - 4.9898064 | - 3.3291519 | | | - 1.9340704 | 0.5211522 9 | | | SEQ ID NO: 28759 |
| | | - 2.7191174 | - 1.5245649 | | | 2.3432463 9 | - 0.1996454 | | | SEQ ID NO: 38017 |
| | | - 1.5712835 | 0.1140830 9 | | | 3.6516580 7 | 0.5859722 3 | | | SEQ ID NO: 38073 |
| | | 0.2973662 9 | - 0.2929861 | | | 3.0331361 8 | 0.1011191 | | | SEQ ID NO: 38076 |
| | | - 0.0118947 | - 1.1537949 | | | 2.3908249 9 | 1.2212076 1 | | | SEQ ID NO: 26298 |
| | | - 1.3179274 | - 1.7164319 | | | 2.9498736 2 | - 0.2800221 | | | SEQ ID NO: 26298 |

### Example 9: Validation of two target proteins as novel G protein-coupled receptor (GPCR) ligands.

This example demonstrates the validation of two novel peptides SEQ ID NO: 30949 and SEQ ID NO: 34229 that act as a GPCR ligand. SEQ ID NO: 30949 blocks CXCR4 and cancer cell migration, making it suitable in the treatment of multiple cancers. Whereas SEQ ID NO: 34229 agonizes C3AR1, a key regulator of immune response and inflammation. The target proteins SEQ ID NO: 30949 and SEQ ID NO: 34229 were treated with *gpcr*MAX, which is a comprehensive panel covering 168 G protein-coupled receptors (GPCRs) from over 60 distinct receptor families. This panel utilizes PathHunter^{®} β-Arrestin technology (Eurofins DiscoverX). PathHunter β-Arrestin GPCR cell lines are engineered to co-express the ProLink^{™} (PK) tagged GPCR and the Enzyme Acceptor (EA) tagged β-Arrestin. Activation of the GPCR-PK induces β-Arrestin-EA recruitment, forcing complementation of the two β-galactosidase enzyme fragments (EA and PK). The resulting functional enzyme hydrolyzes substrate to generate a chemiluminescent signal.

### Materials and Methods:

*Cell Handling:* PathHunter^{®} cell lines were expanded from freezer stocks according to standard procedures. Cells were seeded in a total volume of 20µL into white walled, 384-well microplates and incubated at 37°C for the appropriate time prior to testing.

*Peptide Treatment:* Target proteins SEQ ID NO: 30949, SEQ ID NO: 34229 and irrelevant peptides were synthesized through Fluorenylmethyloxycarbonyl (Fmoc) protection group chemistry using Solid Phase Peptide Synthesis (SPPS). For screening: Target proteins SEQ ID NO: 30949, SEQ ID NO: 34229 and 8 irrelevant peptides were treated on *gpcr*MAX panel at a final top testing concentration of 0.12µM. For hit-confirmation: Target protein SEQ ID NO: 30949 and irrelevant peptides were tested on CXCR4 Human Chemokine GPCR cell-based antagonist arrestin assay at top testing concentrations of 1µM and 0.3µM.

*Assay Design: Agonist Format:* For agonist determination, cells were incubated with sample to induce response. Intermediate dilution of sample stocks was performed to generate 5X sample in assay buffer. 5 µL of 5X sample was added to cells and incubated at 37°C or room temperature for 90 to 180 minutes. Vehicle concentration was 1%. *Antagonist Format:* For antagonist determination, cells were pre-incubated with antagonist followed by agonist challenge at the EC80 concentration. Intermediate dilution of sample stocks was performed to generate 5X sample in assay buffer. 5µL of 5x sample was added to cells and incubated at 37°C or room temperature for 30 minutes. Vehicle concentration was 1%. 5µL of 6X EC80 agonist in assay buffer was added to the cells and incubated at 37°C or room temperature for 90 or 180 minutes.

*CXCR4 Human Chemokine GPCR Cell Based Antagonist Arrestin Assay:* For hit-confirmation, PathHunter^{®} β-Arrestin cell line for CXCR4 human chemokine GPCR antagonist assay was used with CXCL12/SDF-1a as activator and Plerixafor as inhibitor.

*C3aR Human Complement Peptide GPCR Cell Based Agonist Arrestin Assay:* For hit-confirmation, PathHunter^{®} β-Arrestin cell line for C3AR1 human complement peptide GPCR agonist assay was used with C3A Receptor Agonist (Short Fragment) as control activator.

*Signal Detection:* Assay signal was generated through a single addition of 12.5 or 15 µL (50% v/v) of PathHunter^{®} Detection reagent cocktail, followed by a one-hour incubation at room temperature. Microplates were read following signal generation with a PerkinElmer EnvisionTM instrument for chemiluminescent signal detection.

*Data Analysis:* Compound activity was analyzed using CBIS data analysis suite (ChemInnovation, CA). *For agonist mode assays,* percentage activity was calculated using the following formula: % Activity =100% x (mean RLU of test sample - mean RLU of vehicle control) / (mean MAX control ligand - mean RLU of vehicle control). *For antagonist mode assays,* percentage inhibition was calculated using the following formula: % Inhibition = 100% x (1 - (mean RLU of test sample - mean RLU of vehicle control) / (mean RLU of EC80 control - mean RLU of vehicle control)).

*Chemotaxis Assay:* NAMALWA cells (human Burkitt lymphoma cell line, ATCC) were cultured in serum-free ATCC formulated RPMI for 24hr prior to the assay. After starvation, harvest cells and centrifuge at 1,000 x g, for 5 minutes to pellet them. Resuspend the cell in a serum-free media. Using Cell Migration/ Chemotaxis Assay Kit (96-well, 8 µm) from Abcam, add 150 µL of serum-free media containing desired chemoattractant to the bottom chamber. Then add 50,000 cells and desired inhibitor (or peptides) to each well of the top chamber. Place the plate and incubate at 37°C in CO2 incubator for 24 hours. After incubation, add 110 µL of Cell Dye + Cell Dissociation Solution mix to each bottom well and incubate at 37°C in CO2 incubator for an hour. After incubation remove the top chamber and read the plate at Ex/Em = 530/590 nm.

### Results:

Two target peptides, SEQ ID NO: 30949 and SEQ ID NO: 34229 were identified as novel GPCR ligands using Eurofins's gpcrMAX panel. The target peptide SEQ ID NO: 34229 is an agonist of C3AR1, a key anaphylatoxin receptor that plays a critical role in inflammation. The target peptide, SEQ ID NO: 30949 blocked CXCR4, a chemokine receptor involved in cell migration and homing. Additionally, SEQ ID NO: 30949 was able to significantly inhibit the chemotactic migration of human Burkitt lymphoma cells, suggesting its potential as a chemokine for the treatment of cancer (FIG. 7, FIG. 8, FIG. 9, FIG. 10).

The teachings of all patents, published applications and references cited herein are incorporated by reference in their entirety.

While example embodiments have been particularly shown and described, it will be understood by those skilled in the art that various changes in form and details may be made therein without departing from the scope of the embodiments encompassed by the appended claims.

### EMBODIMENTS

1. An agent that comprises and/or modulates the expression or activity of a target protein identified in the Sequence Listing, in Table A, or a variant of the foregoing.
2. The agent of embodiment 1, wherein the agent comprises the target protein.
3. The agent of embodiment 1, wherein the agent modulates the expression of the target protein.
4. The agent of embodiment 2 or 3, wherein the agent modulates the expression of a gene or gene transcript encoding the target protein.
5. The agent of embodiment 1, wherein the agent modulates the activity of the target protein.
6. The agent of any one of embodiments 2-5, comprising a polypeptide, a polynucleotide, or a small molecule.
7. The agent of any one of embodiments 2-6, wherein the agent decreases the expression or activity of the target protein.
8. The agent of embodiment 7, wherein the agent comprises an inhibitor of the target protein.
9. The agent of embodiment 8, wherein the inhibitor is a polypeptide.
10. The agent of embodiment 9, wherein the polypeptide is an antagonist antibody, or an antigen-binding fragment thereof, that binds to the target protein.
11. The agent of embodiment 8, wherein the inhibitor is a polynucleotide.
12. The agent of embodiment 11, wherein the polynucleotide comprises a nucleotide sequence that is complementary to at least a portion of a gene or gene transcript encoding the target protein.
13. The agent of embodiment 11 or 12, wherein the polynucleotide comprises DNA.
14. The agent of embodiment 11 or 12, wherein the polynucleotide comprises RNA.
15. The agent of embodiment 11 or 12, wherein the polynucleotide is a small interfering RNA (siRNA), a short hairpin RNA (shRNA), an antisense DNA, an antisense RNA, a microRNA (miRNA), an antagomir, a guide RNA (gRNA), a locked nucleic acid (LNA) or a morpholino nucleic acid (MNA).
16. The agent of embodiment 8, wherein the inhibitor is a small molecule.
17. The agent of embodiment 16, wherein the small molecule binds to the target protein, thereby decreasing the activity of the target protein.
18. The agent of any one of embodiments 1-6, wherein the agent increases the expression or activity of the target protein.
19. The agent of embodiment 18, wherein the agent is an isolated polypeptide comprising the amino acid sequence of the target protein, or a variant thereof comprising an amino acid sequence having at least 80% sequence identity to the amino acid sequence of the target protein.
20. The agent of embodiment 19, wherein the isolated polypeptide or variant thereof is a recombinant protein or a synthetic protein.
21. The agent of embodiment 18, wherein the agent is a polynucleotide encoding the target protein or a variant thereof having at least 80% sequence identity to the amino acid sequence of the target protein.
22. The agent of embodiment 21, wherein the polynucleotide comprises DNA.
23. The agent of embodiment 21 or 22, wherein the polynucleotide comprises a vector.
24. The agent of embodiment 21, wherein the polynucleotide comprises RNA.
25. The agent of embodiment 24, wherein the RNA is messenger RNA (mRNA) and/or circular RNA (circRNA).
26. The agent of embodiment 18, wherein the agent comprises an activator of the target protein.
27. The agent of embodiment 26, wherein the activator is a polypeptide.
28. The agent of embodiment 27, wherein the polypeptide is an agonist antibody, or an antigen-binding fragment thereof, that binds to the target protein.
29. The agent of embodiment 26, wherein the activator is a small molecule.
30. The agent of embodiment 29, wherein the small molecule binds to the target protein, thereby increasing the activity of the target protein.
31. The agent of any one of embodiments 1-8, 18 and 26, comprising a gene editing system.
32. The agent of embodiment 31, wherein the gene editing system is a CRISPR/Cas system, a transposon-based gene editing system, and a transcription activator-like effector nuclease (TALEN) system.
33. The agent of any one of embodiments 1-32, wherein the target protein is translated from a non-coding RNA.
34. The agent of embodiment 33, wherein the non-coding RNA is a long intergenic non-coding RNA (lincRNA).
35. The agent of any one of embodiments 1-32, wherein the target protein is translated from a non-exonic element in an unprocessed precursor mRNA (pre-mRNA).
36. The agent of embodiment 35, wherein the non-exonic element is an intron in a pre-mRNA.
37. The agent of embodiment 35, wherein the non-exonic element is a 5'-untranslated region (5'-UTR) in a pre-mRNA.
38. The agent of embodiment 35, wherein the non-exonic element is a 3'-untranslated region (3'-UTR) in a pre-mRNA.
39. A pharmaceutical composition comprising the agent of any one of embodiments 1-38, and a pharmaceutically acceptable carrier.
40. The pharmaceutical composition of embodiment 39, wherein the pharmaceutically acceptable carrier is a vector.
41. A method for modulating the expression or activity of a target protein identified in the Sequence Listing, Table A, or a variant of the foregoing in a cell, comprising contacting the cell with the agent of any one of embodiments 1-38 or the pharmaceutical composition of embodiment 39 or 40.
42. The method of embodiment 41, wherein the agent decreases the expression or activity of the target protein in the cell.
43. The method of embodiment 41, wherein the agent increases the expression or activity of the target protein in the cell.
44. The method of any one of embodiments 41-43, wherein the cell is in a subject.
45. The method of embodiment 44, wherein the subject has cancer or a pre-cancerous condition.
46. The method of any one of embodiments 41-45, wherein the agent modulates the expression or activity of the target protein by at least 10%.
47. The method of any one of embodiments 41-46, wherein the agent modulates the expression of a gene encoding the target protein by at least 10%, thereby modulating the expression or activity of the target protein.
48. A method of predicting a likelihood of developing cancer in a subject, comprising quantifying an expression or activity of a target protein identified in the Sequence Listing, Table A, or a variant of the foregoing, in a sample from the subject, wherein the level of expression or activity of the target protein in the sample is indicative of the likelihood of developing cancer in the subject.
49. A method of preparing a sample that is useful for detecting a likelihood of developing cancer in a subject, comprising:
   a) obtaining or having obtained a sample from the subject;
   b) adding a protease inhibitor, a control peptide, a standard peptide, or a combination thereof to the sample to prepare a sample that is useful for detecting a likelihood of developing cancer; and
   c) quantifying an expression or activity of a target protein identified in the Sequence Listing, Table A, or a variant of the foregoing, in the sample prepared in step b).
50. The method of embodiment 48 or 49, further comprising administering to the subject an effective amount of the agent of any one of embodiments 1-38, or the pharmaceutical composition of embodiment 39 or 40 if the subject is predicted to have a likelihood of developing cancer.
51. A method of treating a cancer in a subject in need thereof, comprising administering to the subject an effective amount of the agent of any one of embodiments 1-38, or the pharmaceutical composition of embodiment 39 or 40.
52. The method of any one of embodiments 48-51, wherein the cancer is a solid cancer.
53. The method of any one of embodiments 48-51, wherein the cancer is a hematologic cancer.
54. The method of embodiment 53, wherein the hematologic cancer is a leukemia.
55. The method of embodiment 53, wherein the hematologic cancer is a lymphoma.
56. The method of embodiment 53, wherein the hematologic cancer is multiple myeloma.
57. The method of any one of embodiments 48-51, wherein the cancer is selected from lung cancer, breast cancer, Hodgkin's lymphoma, diffuse large B-cell lymphoma, follicular lymphoma, primary central nervous system lymphoma, chronic lymphocytic leukemia, epithelial ovarian cancer, prostate cancer, squamous cell carcinoma, non-melanoma skin cancer, nasal polyps, basal cell carcinoma, keratinocyte cancer, multiple myeloma, serous invasive ovarian cancer, hepatocellular carcinoma, small cell lung carcinoma, adenocarcinoma, lung adenocarcinoma, non-small cell lung cancer, ovarian cancer or colorectal cancer.
58. A method for identifying an agent that modulates the expression or activity of a target protein identified in the Sequence Listing, Table A, or a variant of the foregoing, comprising:
   a) contacting a protein identified in the Sequence Listing, Table A, or a variant of the foregoing, with an agent; and
   b) determining whether the agent modulates the expression or activity of the target protein,
   wherein a difference in the expression or activity of the target protein that has been contacted with the agent compared to a reference for the expression or activity of the target protein indicates that the agent modulates the expression or activity of the target protein.
59. The method of embodiment 58, wherein a difference of at least 10% in the expression or activity of the target protein that has been contacted with the agent compared to the reference indicates that the agent modulates the expression or activity of the target protein.
60. The method of embodiment 58 or 59, wherein a decrease in the expression or activity of the target protein that has been contacted with the agent compared to the reference indicates the agent inhibits the expression or activity of the target protein.
61. The method of embodiment 58 or 59, wherein an increase in the expression or activity of the target protein compared to the reference indicates the agent activates the expression or activity of the target protein.

## Claims

1. An agent that comprises and/or modulates the expression or activity of a target protein comprising the amino acid sequence of SEQ ID NO: 37997 or a variant thereof.

2. The agent of claim 1, wherein the agent comprises the target protein or wherein the agent modulates the expression of a gene or gene transcript encoding the target protein;
optionally wherein the agent comprises a polypeptide, a polynucleotide, or a small molecule.

3. The agent of claim 1 or 2, wherein the agent increases the expression or activity of the target protein, optionally wherein the agent is:
an isolated polypeptide comprising the amino acid sequence of the target protein, or a variant thereof comprising an amino acid sequence having at least 80% sequence identity to the amino acid sequence of the target protein, optionally wherein the isolated polypeptide or variant thereof is a recombinant protein or a synthetic protein; or
a polynucleotide encoding the target protein or a variant thereof having at least 80% sequence identity to the amino acid sequence of the target protein, optionally wherein the polynucleotide comprises:
DNA and/or a vector; or
RNA, optionally wherein the RNA is messenger RNA (mRNA) or circular RNA (circRNA).

4. The agent of claim 3, wherein the agent comprises an activator of the target protein, optionally wherein the activator is:
a polypeptide, optionally wherein the polypeptide is an agonist antibody, or an antigen-binding fragment thereof, that binds to the target protein; or
a small molecule, optionally wherein the small molecule binds to the target protein,
thereby increasing the activity of the target protein.

5. The agent of any one of claims 1-4, comprising a gene editing system, optionally wherein the gene editing system is a CRISPR/Cas system, a transposon-based gene editing system, and a transcription activator-like effector nuclease (TALEN) system.

6. A pharmaceutical composition comprising the agent of any one of claims 1-5, and a pharmaceutically acceptable carrier, optionally wherein the pharmaceutically acceptable carrier is a vector.

7. An ex vivo method for modulating the expression or activity of a target protein comprising SEQ ID NO: 37997 or a variant thereof in a cell, comprising contacting the cell with the agent of any one of claims 1-5 or the pharmaceutical composition of claim 6;
optionally wherein the agent modulates the expression or activity of the target protein by at least 10%, optionally wherein the agent modulates the expression of a gene encoding the target protein by at least 10%, thereby modulating the expression or activity of the target protein.

8. The method of claim 7, wherein the agent increases the expression or activity of the target protein in the cell.

9. A method of predicting a likelihood of developing cancer in a subject, comprising quantifying an expression or activity of a target protein comprising SEQ ID NO: 37997 or a variant thereof in a sample from the subject, wherein the level of expression or activity of the target protein in the sample is indicative of the likelihood of developing cancer in the subject;
optionally wherein the method further comprises use of an effective amount of the agent of any one of claims 1-5, or the pharmaceutical composition of claim 6 for administering to the subject, optionally wherein the subject is predicted to have a likelihood of developing cancer.

10. A method of preparing a sample that is useful for detecting a likelihood of developing cancer in a subject, comprising:
a) obtaining or having obtained a sample from the subject;
b) adding a protease inhibitor, a control peptide, a standard peptide, or a combination thereof to the sample to prepare a sample that is useful for detecting a likelihood of developing cancer; and
c) quantifying an expression or activity of a target protein comprising SEQ ID NO: 37997 or a variant thereof in the sample prepared in step b);
optionally wherein the method further comprises use of an effective amount of the agent of any one of claims 1-5, or the pharmaceutical composition of claim for administering to the subject, optionally wherein the subject is predicted to have a likelihood of developing cancer.

11. The agent of any one of claims 1-5, or the pharmaceutical composition of claim 8, for use in a method of treating a cancer in a subject in need thereof.

12. The method of any one of claims 9 and 10, or the agent or pharmaceutical composition for use of claim 11, wherein the cancer is a solid cancer; optionally wherein the solid cancer is selected from lung cancer, breast cancer, epithelial ovarian cancer, prostate cancer, squamous cell carcinoma, non-melanoma skin cancer, nasal polyps, basal cell carcinoma, keratinocyte cancer, serous invasive ovarian cancer, hepatocellular carcinoma, small cell lung carcinoma, adenocarcinoma, lung adenocarcinoma, non-small cell lung cancer, and ovarian cancer or colorectal cancer.

13. The method of any one of claims 9 and 10, or the agent or pharmaceutical composition for use of claim 11 wherein the cancer is a hematologic cancer, optionally wherein the hematologic cancer is a leukemia, a lymphoma, or multiple myeloma; and
optionally wherein the hematologic cancer is selected from Hodgkin's lymphoma, diffuse large B-cell lymphoma, follicular lymphoma, primary central nervous system lymphoma, and chronic lymphocytic leukemia.

14. A method for identifying an agent that modulates the expression or activity of a target protein comprising SEQ ID NO: 37997 or a variant thereof, comprising:
a) contacting the protein with the agent; and
b) determining whether the agent modulates the expression or activity of the target protein,
wherein a difference in the expression or activity of the target protein that has been contacted with the agent compared to a reference for the expression or activity of the target protein indicates that the agent modulates the expression or activity of the target protein;
optionally wherein a difference of at least 10% in the expression or activity of the target protein that has been contacted with the agent compared to the reference indicates that the agent modulates the expression or activity of the target protein; and
optionally wherein a decrease in the expression or activity of the target protein that has been contacted with the agent compared to the reference indicates the agent inhibits the expression or activity of the target protein, or an increase in the expression or activity of the target protein compared to the reference indicates the agent activates the expression or activity of the target protein.

15. An ex vivo method of increasing basal apoptosis levels and/or decreasing proliferation of cancer cells, comprising contacting cancer cells with the agent of any one of claims 1-5 or the pharmaceutical composition of claim 6.
